# EUROPEAN PATENT SPECIFICATION

(11) **EP 4 319 670 B1**
(45) Date of publication and mention of the grant of the patent: **06.08.2025**
(21) Application number: 22715759.1
(22) Date of filing: 05.04.2022
(51) Int. Cl.: A61B 18/12, A61B 18/14, A61B 18/00, A61B 34/20

(54) **ENT INSTRUMENT WITH RF ELECTRODES ON WIRE FRAME**
ENT-INSTRUMENT MIT HF-ELEKTRODEN AUF EINEM DRAHTRAHMEN
INSTRUMENT ORL DOTÉ D'ÉLECTRODES RF SUR UN CADRE MÉTALLIQUE

(30) Priority: 06.04.2021 US 202163171111 P; 28.03.2022 US 202217705440
(43) Date of publication of application: 14.02.2024
(73) Proprietor: Acclarent, Inc., Irvine, CA 92618 (US); BIOSENSE WEBSTER (ISRAEL) LTD., 2066717 Yokneam (IL)
(72) Inventor: PALUSHI, Jetmir, Irvine, California 92620 (US); SALAZAR, Henry F., Irvine, California 92618 (US); ABDELWAHED, Hany, Irvine, California 92618 (US); BASU, Shubhayu, Irvine, California 92618 (US); FANG, Itzhak, Irvine, California 92614 (US); VANOSDOLL, Madison K., Cincinnati, Ohio 45242 (US); INSLEY, John W., Irvine, California 92618 (US)
(74) Representative: Carpmaels & Ransford LLP
(86) International application number: PCT/IB2022/053149
(87) International publication number: WO 2022/214953

(56) References cited:
- US-A1- 2013 096 554
- US-A1- 2015 257 824
- US-A1- 2019 021 620
- US-A1- 2019 239 954

## Description

### BACKGROUND

Rhinitis is a medical condition that presents as irritation and inflammation of the mucous membrane within the nasal cavity. The inflammation results in the generation of excessive amounts of mucus, which can cause runny nose, nasal congestion, sneezing, and/or post-nasal drip. Allergenic rhinitis is an allergic reaction to environmental factors such as airborne allergens, while non-allergenic (or "vasomotor") rhinitis is a chronic condition that presents independently of environmental factors. Conventional treatments for rhinitis include antihistamines, topical or systemic corticosteroids, and topical anticholinergics, for example.

For cases of intractable rhinitis in which the symptoms are severe and persistent, an additional treatment option is the surgical removal of a portion of the vidian (or "pterygoid") nerve - a procedure known as vidian neurectomy. The theoretical basis for vidian neurectomy is that rhinitis is caused by an imbalance between parasympathetic and sympathetic innervation of the nasal cavity, and the resultant over stimulation of mucous glands of the mucous membrane. Vidian neurectomy aims to disrupt this imbalance and reduce nasal mucus secretions via surgical treatment of the vidian nerve. However, in some instances, vidian neurectomy can cause collateral damage to the lacrimal gland, which is innervated by the vidian nerve. Such damage to the lacrimal gland may result in long-term health complications for the patient, such as chronic dry eye. Posterior nasal neurectomy, or surgical removal of a portion of the posterior nasal nerves, may be an effective alternative to vidian neurectomy for treating intractable rhinitis.

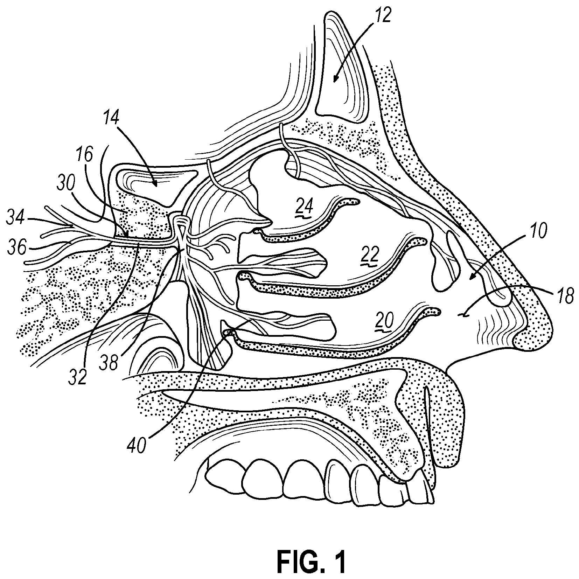

FIG. 1 depicts a left sagittal view of a portion of a patient's head, showing the nasal cavity (10), the frontal sinus (12), the sphenoid sinus (14), and the sphenoid bone (16). The nasal cavity (10) is bounded laterally by the nasal wall (18), which includes an inferior turbinate (20), a middle turbinate (22), and a superior turbinate (24). The vidian nerve (32) resides within the vidian (or "pterygoid") canal (30), which is defined in part by the sphenoid bone (16) and is located posterior to the sphenoid sinus (14), approximately in alignment with the middle turbinate (22). The vidian nerve (32) is formed at its posterior end by the junction of the greater petrosal nerve (34) and the deep petrosal nerve (36); and joins at its anterior end with the pterygopalatine ganglion (38), which is responsible for regulating blood flow to the nasal mucosa. The posterior nasal nerves (40) join with the pterygopalatine ganglion (38) and extend through the region surrounding the inferior turbinate (20).

US 2019/0239954 A1 describes devices for therapeutic nasal neuromodulation and associated systems and methods are disclosed herein. A disclosed system for therapeutic neuromodulation in a nasal region includes a shaft and a therapeutic element at a distal portion of the shaft. The shaft can locate the distal portion intraluminally at a target site inferior to a patient's sphenopalatine foramen. The therapeutic element can include an energy delivery element configured to therapeutically modulate postganglionic parasympathetic nerves at microforamina of a palatine bone of the human patient for the treatment of rhinitis or other indications. In other embodiments, the therapeutic element can be configured to therapeutically modulate nerves that innervate the frontal, ethmoidal, sphenoidal, and maxillary sinuses for the treatment of chronic sinusitis. US 2015/0257824 Al1 describes a neuromodulation catheter that includes an elongate shaft and a neuromodulation element operably connected to the shaft. In embodiments, the neuromodulation element includes an elongate support member and a plurality of radial-expansion members operably connected to the support member in a distal-to-proximal sequence. In an embodiment, Individual radial-expansion members partially overlap longitudinally.

While instruments and methods for performing vidian neurectomies, posterior nasal neurectomies, and turbinate reductions are known, it is believed that no one prior to the inventors has made or used the invention described in the appended claims.

### BRIEF DESCRIPTION OF THE DRAWINGS

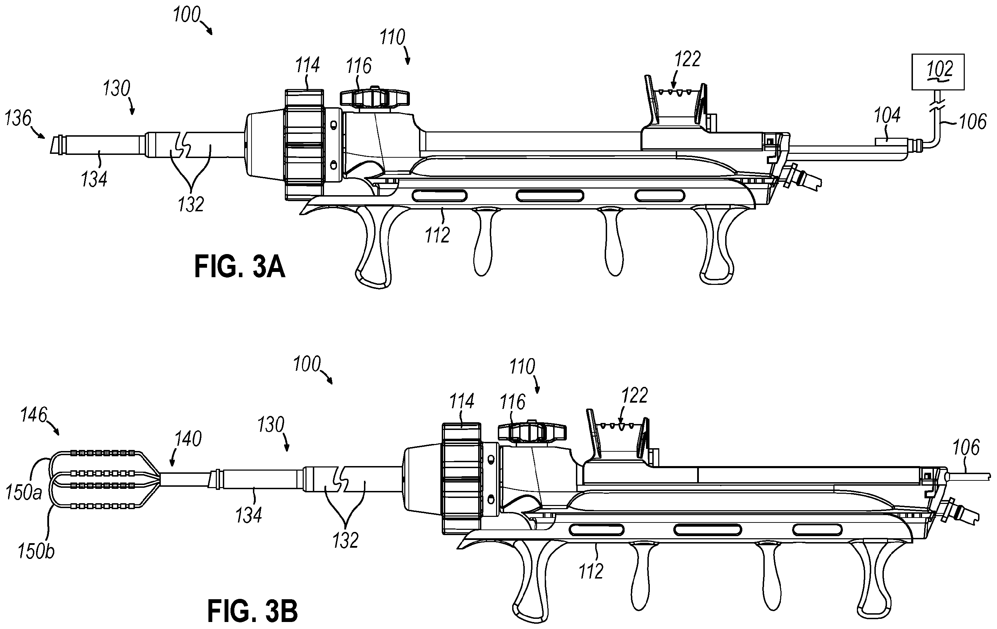

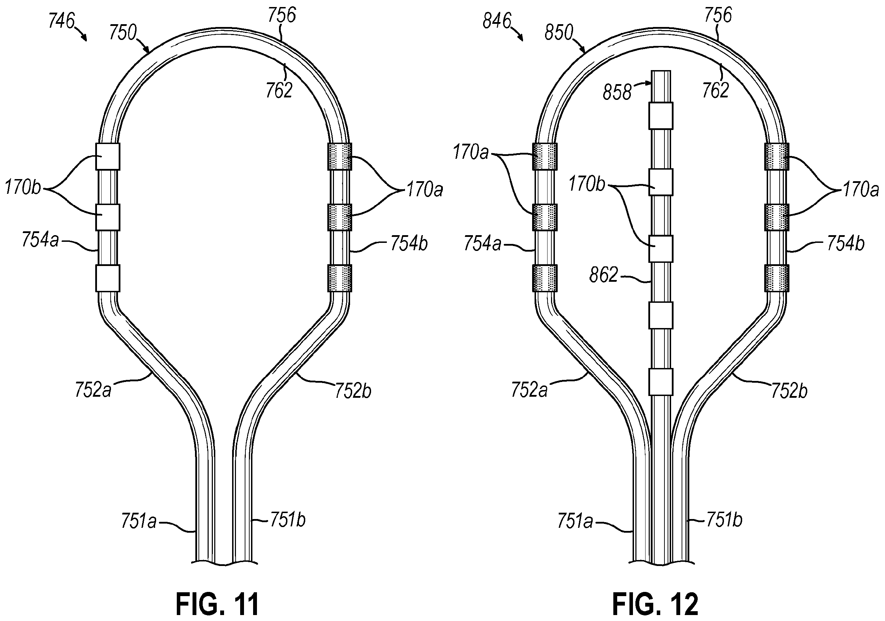

The drawings and detailed description that follow are intended to be merely illustrative and are not intended to limit the scope of the invention as contemplated by the inventors. Figures 3 to 10 and 19 illustrate aspects of the claimed invention, as defined by the appended claims. Figures 11 to 18 illustrate embodiments not defined by the wording of the appended claims, but are helpful for understanding of aspects of the invention.
FIG. 1 depicts a left sagittal view of a portion of a patient's head, showing details of certain paranasal sinuses and nerves, including the vidian nerve and the posterior nasal nerve;
FIG. 2 depicts a schematic view of an exemplary surgery navigation system being used on a patient seated in an exemplary medical procedure chair;
FIG. 3A depicts a side elevational view of an exemplary instrument that may be used to perform an ablation procedure in a nasal cavity, showing an energy catheter of the instrument in a proximal retracted position relative to a shaft assembly of the instrument;
FIG. 3B depicts a side elevational view of the instrument of FIG. 3A, showing the energy catheter of the instrument in a distal extended position relative to the shaft assembly of the instrument;
FIG. 4 depicts a perspective view of a distal portion of the energy catheter shown in FIG. 3A;
FIG. 5 depicts a perspective view of a ring electrode of the energy catheter shown in FIG. 3A
FIG. 6 depicts a perspective view of a distal portion of another exemplary energy catheter for use with the instrument of FIG. 3A;
FIG. 7 depicts a perspective view of a distal portion of another exemplary energy catheter for use with the instrument of FIG. 3A;
FIG. 8 depicts a perspective view of a distal portion of another exemplary energy catheter for use with the instrument of FIG. 3A;
FIG. 9 depicts a perspective view of a distal portion of another exemplary energy catheter for use with the instrument of FIG. 3A;
FIG. 10 depicts a perspective view of a distal portion of another exemplary energy catheter for use with the instrument of FIG. 3A;
FIG. 11 depicts a perspective view of a distal portion of another exemplary end effector for use with the instrument of FIG. 3A;
FIG. 12 depicts a perspective view of a distal portion of another exemplary end effector for use with the instrument of FIG. 3A;
FIG. 13 depicts a perspective view of a distal portion of another exemplary end effector for use with the instrument of FIG. 3A;
FIG. 14 depicts a perspective view of a distal portion of another exemplary end effector for use with the instrument of FIG. 3A;
FIG. 15A depicts a perspective view of a distal portion of another exemplary end effector for use with the instrument of FIG. 3A, showing a plurality of ring electrodes selectively attached to respective longitudinal segments of the end effector;
FIG. 15B depicts a perspective view of the distal portion of the end effector of FIG. 15A, showing a portion of the plurality of ring electrodes selectively removed from the respective longitudinal segments of the end effector;
FIG. 16A depicts a perspective view of a distal portion of another exemplary end effector for use with the instrument of FIG. 3A, showing a plurality of ring electrodes selectively attached to respective arcuate segments of the end effector;
FIG. 16B depicts a perspective view of the distal portion of the end effector of FIG. 16A, showing a portion of the plurality of ring electrodes selectively removed from the respective arcuate segments of the end effector;
FIG. 17 depicts a perspective view of a distal portion of another exemplary end effector for use with the instrument of FIG. 3A;
FIG. 18 depicts a perspective view of a distal portion of another exemplary end effector for use with the instrument of FIG. 3A;
FIG. 19A depicts a left sagittal view of a portion of a patient's head, showing insertion of a distal portion of the instrument of FIG. 3A into the patient's nasal cavity in the region of a posterior nasal nerve, with the energy catheter in the proximal retracted position relative to the shaft assembly; and
FIG. 19B depicts a left sagittal view of the portion of the patient's head with the distal portion of the instrument of FIG. 3A inserted in the patient's nasal cavity in the region of the posterior nasal nerve, with the energy catheter in the distal extended position relative to the shaft assembly to deliver RF energy to a portion of the posterior nasal nerve to thereby ablate the nerve portion.

### DETAILED DESCRIPTION

The invention is defined by the appended claims. The following description of certain examples of the invention should not be used to limit the scope of the present invention. Other examples, features, aspects, embodiments, and advantages of the invention will become apparent to those skilled in the art from the following description. As will be realized, the invention is capable of other different and obvious aspects, all without departing from the invention, which is defined by the appended claims. Accordingly, the drawings and descriptions should be regarded as illustrative in nature and not restrictive.

For clarity of disclosure, the terms "proximal" and "distal" are defined herein relative to a surgeon, or other operator, grasping a surgical instrument having a distal surgical end effector. The term "proximal" refers to the position of an element arranged closer to the surgeon, and the term "distal" refers to the position of an element arranged closer to the surgical end effector of the surgical instrument and further away from the surgeon. Moreover, to the extent that spatial terms such as "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

As used herein, the terms "about" and "approximately" for any numerical values or ranges indicate a suitable dimensional tolerance that allows the part or collection of components to function for its intended purpose as described herein.

### I. Exemplary Image Guided Surgery Navigation System

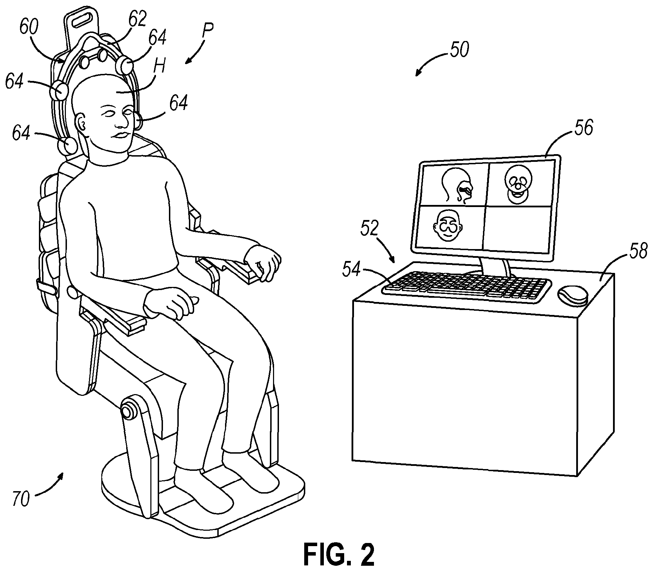

When performing a medical procedure within a head (H) of a patient (P), it may be desirable to have information regarding the position of an instrument within the head (H) of the patient (P), particularly when the instrument is in a location where it is difficult or impossible to obtain an endoscopic view of a working element of the instrument within the head (H) of the patient (P). FIG. 2 shows an exemplary IGS navigation system (50) enabling an ENT procedure to be performed using image guidance. In addition to or in lieu of having the components and operability described herein IGS navigation system (50) may be constructed and operable in accordance with at least some of the teachings of U.S. Pat. No. 7,720,521, entitled "Methods and Devices for Performing Procedures within the Ear, Nose, Throat and Paranasal Sinuses," issued May 18, 2010; and U.S. Pat. Pub. No. 2014/0364725, entitled "Systems and Methods for Performing Image Guided Procedures within the Ear, Nose, Throat and Paranasal Sinuses," published December 11, 2014, now abandoned.

IGS navigation system (50) of the present example comprises a field generator assembly (60), which comprises set of magnetic field generators (64) that are integrated into a horseshoe-shaped frame (62). Field generators (64) are operable to generate alternating magnetic fields of different frequencies around the head (H) of the patient (P). An instrument, such as any of the RF ablation instruments described below, may be inserted into the head (H) of the patient (P). Such an instrument may be a standalone device or may be positioned on an end effector. In the present example, frame (62) is mounted to a chair (70), with the patient (P) being seated in the chair (70) such that frame (62) is located adjacent to the head (H) of the patient (P). By way of example only, chair (70) and/or field generator assembly (60) may be configured and operable in accordance with at least some of the teachings of U.S. Pat. No. 10,561,370, entitled "Apparatus to Secure Field Generating Device to Chair," Issued February 18, 2020.

IGS navigation system (50) of the present example further comprises a processor (52), which controls field generators (64) and other elements of IGS navigation system (50). For instance, processor (52) is operable to drive field generators (64) to generate alternating electromagnetic fields; and process signals from the instrument to determine the location of a navigation sensor in the instrument within the head (H) of the patient (P). Processor (52) comprises a processing unit (e.g., a set of electronic circuits arranged to evaluate and execute software instructions using combinational logic circuitry or other similar circuitry) communicating with one or more memories. Processor (52) of the present example is mounted in a console (58), which comprises operating controls (54) that include a keypad and/or a pointing device such as a mouse or trackball. A physician uses operating controls (54) to interact with processor (52) while performing the surgical procedure.

While not shown, the instrument may include a navigation sensor that is responsive to positioning within the alternating magnetic fields generated by field generators (64). A coupling unit (not shown) may be secured to the proximal end of the instrument and may be configured to provide communication of data and other signals between console (58) and the instrument. The coupling unit may provide wired or wireless communication of data and other signals.

In some versions, the navigation sensor of the instrument may comprise at least one coil at or near the distal end of the instrument. When such a coil is positioned within an alternating electromagnetic field generated by field generators (64), the alternating magnetic field may generate electrical current in the coil, and this electrical current may be communicated along the electrical conduit(s) in the instrument and further to processor (52) via the coupling unit. This phenomenon may enable IGS navigation system (50) to determine the location of the distal end of the instrument within a three-dimensional space (i.e., within the head (H) of the patient (P), etc.). To accomplish this, processor (52) executes an algorithm to calculate location coordinates of the distal end of the instrument from the position related signals of the coil(s) in the instrument.

Processor (52) uses software stored in a memory of processor (52) to calibrate and operate IGS navigation system (50). Such operation includes driving field generators (64), processing data from the instrument, processing data from operating controls (54), and driving display screen (56). In some implementations, operation may also include monitoring and enforcement of one or more safety features or functions of IGS navigation system (50). Processor (52) is further operable to provide video in real time via display screen (56), showing the position of the distal end of the instrument in relation to a video camera image of the patient's head (H), a CT scan image of the patient's head (H), and/or a computer-generated three-dimensional model of the anatomy within and adjacent to the patient's nasal cavity. Display screen (56) may display such images simultaneously and/or superimposed on each other during the surgical procedure. Such displayed images may also include graphical representations of instruments that are inserted in the patient's head (H), such that the operator may view the virtual rendering of the instrument at its actual location in real time. By way of example only, display screen (56) may provide images in accordance with at least some of the teachings of U.S. Pat. No. 10,463,242, entitled "Guidewire Navigation for Sinuplasty," issued November 5, 2019. In the event that the operator is also using an endoscope, the endoscopic image may also be provided on display screen (56).

The images provided through display screen (56) may help guide the operator in maneuvering and otherwise manipulating instruments within the patient's head (H). It should also be understood that other components of a surgical instrument and other kinds of surgical instruments, as described below, may incorporate a navigation sensor like the navigation sensor described above.

### II. Exemplary RF Ablation Instruments with Expandable Electrode Assemblies Having Resilient Support Wires

In some instances, it may be desirable to provide an RF ablation instrument having at least one electrode assembly that is selectively actuatable between non-expanded and expanded states to facilitate effective and safe RF ablation of a nasal nerve, such as the posterior nasal nerve (40), as an alternative to a vidian neurectomy procedure. Such ablation instruments may facilitate insertion into a nasal cavity to reach a target region while the ablation members are in a non-expanded state; and promote full contact with targeted tissue when the ablation members are in the expanded state. The exemplary RF ablation instrument (100) described below may function in such a manner. While the examples provided below are discussed in the context of posterior nasal nerve (40) ablation, RF ablation instrument (100) may be used to ablate tissue in various other regions within the ear, nose, or throat of a patient. Other suitable ways in which RF ablation instrument (100) may be used will be apparent to those skilled in the art in view of the teachings herein.

### A. Exemplary RF Ablation Instrument with Energy Catheter Having Double Loop Electrode Assemblies with Single Polarities

FIGS. 3A-5 show an example of an instrument (100) that may be used to deliver RF energy to tissue. For instance, instrument (100) may be used to ablate a nerve (e.g., the posterior nasal nerve (40)); ablate a turbinate (e.g., any of turbinates (20, 22, 24)); or ablate, electroporate (e.g., to promote absorption of therapeutic agents, etc.), or apply resistive heating to any other kind of anatomical structure in the head of a patient. Instrument (100) of this example includes a handle assembly (110), a shaft assembly (130), and an energy catheter (140) with an elongate end effector (146) having a first loop electrode assembly (150a) and a second loop electrode assembly (150b). Instrument (100) is coupled with an RF generator (102) via an energy catheter connector (104) positioned at a proximal end of energy catheter (140). Energy catheter connector (104) may selectively couple to a distal end of a cable (106) having a proximal end coupled to RF generator (102). In other versions, cable (106) may be integrated directly with energy catheter (140). In any event, RF generator (102) is operable to generate RF electrosurgical energy for delivery to tissue via electrodes (170a, 170b) as will be described in greater detail below. Such RF electrosurgical energy may be delivered in a monopolar modality or a bipolar modality.

Handle assembly (110) of this example includes a body (112) and a slider (122). Body (112) is sized and configured to be grasped and operated by a single hand of an operator, such as via a power grip, a pencil grip, or any other suitable kind of grip. Slider (122) is operable to translate longitudinally relative to body (112). Slider (122) is coupled with energy catheter (140) and is thus operable to translate energy catheter (140) with loop electrode assemblies (150a, 150b) longitudinally as will be described in greater detail below. The transition from FIG. 3A to FIG. 3B shows loop electrode assemblies (150a, 150b) being driven by slider (122) from a proximal position (FIG. 3A) to a distal position (FIG. 3B).

Shaft assembly (130) of the present example includes a rigid portion (132), a flexible portion (134) distal to rigid portion (132), and an open distal end (136). A pull-wire (not shown) is coupled with flexible portion (134) and with a deflection control knob (116) of handle assembly (110). Deflection control knob (116) is rotatable relative to body (112), about an axis that is perpendicular to the longitudinal axis of shaft assembly (130), to selectively retract the pull-wire proximally. As the pull-wire is retracted proximally, flexible portion (134) bends and thereby deflects distal end (136) laterally away from the longitudinal axis of rigid portion (132). Deflection control knob (116), the pull-wire, and flexible portion (134) thus cooperate to impart steerability to shaft assembly (130). By way of example only, such steerability of shaft assembly (130) may be provided in accordance with at least some of the teachings of U.S. Pub. No. 2021/0361912, entitled "Shaft Deflection Control Assembly for ENT Guide Instrument," published November 25, 2021. Other versions may provide some other kind of user input feature to drive steering of flexible portion (134), instead of deflection control knob (116). In some versions, flexible portion (134) may be formed of nitinol. In addition, or alternatively, open distal end (136) may be configured to dissect tissue. In some alternative versions, deflection control knob (116) is omitted, and flexible portion (134) is malleable. In still other versions, the entire length of shaft assembly (130) is rigid.

Shaft assembly (130) is also rotatable relative to handle assembly (110), about the longitudinal axis of rigid portion (132). Such rotation may be driven via rotation control knob (114), which is rotatably coupled with body (112) of handle assembly (110). Alternatively, shaft assembly (130) may be rotated via some other form of user input; or may be non-rotatable relative to handle assembly (110). It should also be understood that the example of handle assembly (110) described herein is merely an illustrative example. Shaft assembly (130) may instead be coupled with any other suitable kind of handle assembly or other supporting body.

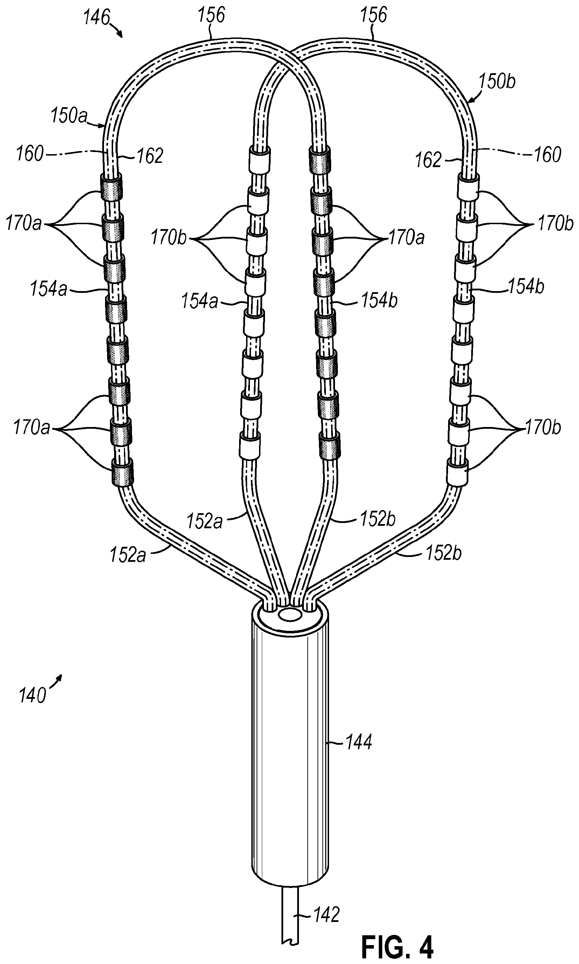

As best seen in FIG. 4, energy catheter (140) of the present example includes a body (e.g., a shaft) having a proximal body portion (142) and a distal body portion (144), with elongate end effector (146) having loop electrode assemblies (150a, 150b) extending distally from distal body portion (144). In this regard, and in embodiments not defined by the wording of the appended claims, loop electrode assemblies (150a, 150b) extend along respective planes that are each parallel to and positioned along the longitudinal axis of distal body portion (144) and/or the longitudinal axis of shaft assembly (130). In some other versions, and in accordance with the invention, the planes defined by loop electrode assemblies (150a, 150b) are parallel with, yet laterally offset from, the longitudinal axis of distal body portion (144) and/or the longitudinal axis of shaft assembly (130). Each loop electrode assembly (150a, 150b) includes first and second outwardly splayed segments (152a, 152b) coupled to a distal end of distal body portion (144), first and second longitudinal segments (154a, 154b) extending distally from distal ends of respective outwardly splayed segments (152a, 152b), and an arcuate transverse segment (156) extending between distal ends of longitudinal segments (154a, 154b).

In the present example, loop electrode assemblies (150a, 150b) each define a bulbous generally elliptical or oval shape such that end effector (146) is generally elongate (e.g., having a length greater than its width) at least when loop electrode assemblies (150a, 150b) are in the distal position (i.e., exposed relative to shaft assembly (130)). Alternatively, loop electrode assemblies (150a, 150b) may define a different shape in other versions. Moreover, while loop electrode assemblies (150a, 150b) are each generally symmetric in this example, loop electrode assemblies (150a, 150b) may be asymmetric if desired. Loop electrode assemblies (150a, 150b) of the present example are also each at least partially formed of a resilient material (e.g., nitinol, etc.) as described below, such that loop electrode assemblies (150a, 150b) are each resiliently biased to form the respective generally bulbous shapes, though loop electrode assemblies (150a, 150b) are each configured to deform laterally, inwardly, and otherwise. For instance, loop electrode assemblies (150a, 150b) may deform when loop electrode assemblies (150a, 150b) are pressed against tissue, when loop electrode assemblies (150a, 150b) are contained within flexible portion (134) of shaft assembly (130), etc.

In the example shown, and in accordance with the invention, first and second loop electrode assemblies (150a, 150b) at least partially overlap each other. More particularly, first outwardly splayed segment (152a) of second loop electrode assembly (150b) connects to distal body portion (144) at a location between the locations at which outwardly splayed segments (152a, 152b) of first loop electrode assembly (150a) connect to distal body portion (144); and second outwardly splayed segment (152b) of first loop electrode assembly (150a) connects to distal body portion (144) at a location between the locations at which outwardly splayed segments (152a, 152b) of second loop electrode assembly (150b) connect to distal body portion (144). In other versions, not defined by the wording of the appended claims, first and second loop electrode assemblies (150a, 150b) may not overlap each other. For example, first outwardly splayed segment (152a) of second loop electrode assembly (150b) may connect to distal body portion (144) at a location between the locations at which second outwardly splayed segments (152b) of loop electrode assemblies (150a) connect to distal body portion (144), and second outwardly splayed segment (152b) of first loop electrode assembly (150a) may connect to distal body portion (144) at a location between the locations at which first outwardly splayed segments (152a) of loop electrode assemblies (150b) connect to distal body portion (144).

As further shown in FIG. 4, loop electrode assemblies (150a, 150b) each include a flexible support wire (160) extending along each of the corresponding segments (152a, 152b, 154a, 154b, 156). Each support wire (160) is resiliently biased to define the generally bulbous shape of the respective loop electrode assembly (150a, 150b). Support wires (160) may each be resiliently biased to define any other predetermined geometry of the respective loop electrode assembly (150a, 150b). In some versions, support wires (160) may each be metallic. For example, support wires (160) may each be formed of nitinol. Loop electrode assemblies (150a, 150b) of the present example each further include an electrically insulative coating or sleeve (162) extending over the respective support wire (160), for reasons discussed below.

Loop electrode assemblies (150a, 150b) each also include a plurality of energy application elements in the form of first and second ring electrodes (170a, 170b) operatively secured to the corresponding support wire (160). Ring electrodes (170a) are coaxially aligned with each other and are longitudinally spaced apart from each other along respective first and second longitudinal segments (154a, 154b) of loop electrode assembly (150a). Similarly, ring electrodes (170b) are coaxially aligned with each other and are longitudinally spaced apart from each other along respective first and second longitudinal segments (154a, 154b) of loop electrode assembly (150b).

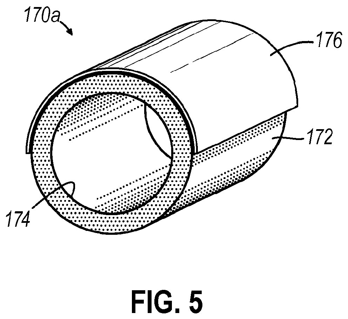

As shown in FIG. 5, each first ring electrode (170a) includes an electrically conductive cylinder (172) defining a bore (174) for receiving the corresponding support wire (160) and/or other components of the respective loop electrode assembly (150a, 150b). First ring electrodes (170a) may each also include an electrically insulative coating or layer (176) applied partially over an outer cylindrical surface of the corresponding conductive cylinder (172). For example, insulative layer (176) may be applied over approximately one-half of the outer cylindrical surface of conductive cylinder (172). Insulative layer (176) may include one or more of a non-conductive glue, spray coating, polytetrafluoroethylene (PTFE), or polyether block amide (PEBA), for example. In this manner, insulative layer (176) may be configured to protect tissue on the side of first ring electrode (170a) faced by insulative layer (176) from RF energy applied by the corresponding conductive cylinder (172) of first ring electrode (172a), as described below. Each second ring electrode (170b) may be formed similarly to first ring electrodes (170a); and may likewise include cylinder (172) with bore (174) and/or insulative layer (176). While eight ring electrodes (170a, 170b) are shown positioned along each longitudinal segment (154a, 154b) in FIG. 4, any other suitable number of ring electrodes (170a, 170b) may be provided.

With continuing reference to FIG. 4, each insulative sleeve (162) extends along each of the corresponding segments (152a, 152b, 154a, 154b, 156) and between each of the respective ring electrodes (170a, 170b); with ring electrodes (170a, 170b) being left exposed. In this manner, insulative sleeve (162) may prevent short circuiting within shaft assembly (130) and/or between adjacent ring electrodes (170a, 170b). In some versions, sleeve (162) of each loop electrode assembly (150a, 150b) may be received within the respective bores (174) of the corresponding ring electrodes (170a, 170b).

The conductive cylinder (172) of each ring electrode (170a, 170b) is coupled with a corresponding one or more wire(s), trace(s), and/or other conductive element(s) that electrically couple conductive cylinders (172) with RF generator (102). In some versions, conductive cylinders (172) are each electrically coupled with RF generator (102) via a conductive element in the form of the corresponding support wire (160). Alternatively, conductive cylinders (172) may each be electrically coupled with RF generator (102) via one or more conductive elements that are separate and distinct from the corresponding support wire (160). In such cases, the conductive element(s) may each extend along, and be electrically insulated from, the corresponding support wire (160). In this regard, each sleeve (162) may define a respective passageway (not shown) for receiving the corresponding support wire (160) and/or conductive element(s). In any event, first ring electrodes (170a) are each configured to apply RF energy at a first polarity; while second ring electrodes (170b) are each configured to apply RF energy at a second polarity. Ring electrodes (170a, 170b) thus are operable to apply bipolar RF energy to tissue contacting ring electrodes (170a, 170b), with first ring electrodes (170a) serving as active electrodes and second ring electrodes (170b) serving as return electrodes.

**In** the example shown, first ring electrodes (170a) are positioned along first and second longitudinal segments (154a, 154b) of first loop electrode assembly (150a), and second ring electrodes (170b) are positioned along first and second longitudinal segments (154a, 154b) of second loop electrode assembly (150b), such that first and second loop electrode assemblies (150a, 150b) are configured to apply RF energy at first and second polarities, respectively. Thus, loop electrode assemblies (150a, 150b) may cooperate with each other to apply bipolar RF energy to tissue. In some versions, conductive cylinders (172) of first ring electrodes (170a) of first loop electrode assembly (150a) may be electrically coupled with RF generator (102) via a single conductive element (e.g., the corresponding support wire (160) of first loop electrode assembly (150a)), and conductive cylinders (172) of second ring electrodes (170b) of second loop electrode assembly (150b) may be electrically coupled with RF generator (102) via a single conductive element (e.g., the corresponding support wire (160) of second loop electrode assembly (150b)). Due to this arrangement of ring electrodes (170a, 170b) and the overlapping relationship of first loop electrode assembly (150a) with second loop electrode assembly (150b), the polarities of ring electrodes (170a, 170b) may alternate in a circumferential direction.

It will be appreciated that ring electrodes (170a, 170b) may be positioned along longitudinal segments (154a, 154b) of loop electrode assemblies (150a, 150b) in any suitable arrangement, such as with first ring electrodes (170a) positioned along first longitudinal segments (154a) of loop electrode assemblies (150a, 150b) and second ring electrodes (170b) positioned along second longitudinal segments (154b) of loop electrode assemblies (150a, 150b), or vice versa, such that first and second loop electrode assemblies (150a, 150b) may independently apply bipolar RF energy to tissue, as described in greater detail below. In other versions, first and second ring electrodes (170a, 170b) may be positioned along each longitudinal segment (154a, 154b) of both loop electrode assemblies (150a, 150b), such as in an alternating arrangement with adjacent first and second ring electrodes (170a, 170b) electrically isolated from each other. Other suitable ways in which polarities may be allocated among loop electrode assemblies (150a, 150b) will be apparent to those skilled in the art in view of the teachings herein. In addition, or alternatively, at least a portion of ring electrodes (170a, 170b) may be selectively removable from the respective longitudinal segments (154a, 154b), as described below.

As shown in FIGS. 3A and 3B, energy catheter (140) is selectively actuatable via slider (122) between a proximal retracted position (FIG. 3A) and a distal extended position (FIG. 3B) relative to shaft assembly (130). In the exemplary proximal retracted position of FIG. 3A, end effector (146) including loop electrode assemblies (150a, 150b) is housed within flexible portion (134) of shaft assembly (130) in a non-expanded state. In this regard, flexible portion (134) may have an internal cross dimension sized to constrict loop electrode assemblies (150a, 150b) to the non-expanded state. Thus, flexible portion (134) may radially compress loop electrode assemblies (150a, 150b) and prevent loop electrode assemblies (150a, 150b) from radially expanding, or otherwise restrict radial expansion of loop electrode assemblies (150a, 150b), when energy catheter (140) is in the proximal retracted position. In the exemplary distal extended position of FIG. 3B, end effector (146) including loop electrode assemblies (150a, 150b) is exposed from flexible portion (134) of shaft assembly (130) so as to extend distally beyond distal end (136). Thus, flexible portion (134) may permit loop electrode assemblies (150a, 150b) to resiliently expand outwardly from a central longitudinal axis to an expanded state in response to energy catheter (140) transitioning to the distal extended position.

As shown in FIG. 3B, loop electrode assemblies (150a, 150b) are each automatically expandable outwardly from the non-expanded state to the expanded state, due to the resilient bias of loop electrode assemblies (150a, 150b), so as to extend radially outwardly beyond distal body portion (144) in response to actuation of energy catheter (140) to the distal extended position. Thus, energy catheter (140) is configured to be actuated from the proximal retracted position to the distal extended position; and in response thereto, loop electrode assemblies (150a, 150b) are configured to be automatically expanded from the non-expanded state to the expanded state to urge loop electrode assemblies (150a, 150b) and, more particularly, ring electrodes (170a, 170b) into electrical contact with the target tissue of a patient following insertion of the distal end of RF ablation instrument (100) into the nasal cavity (10).

During use of loop electrode assemblies (150a, 150b), when loop electrode assemblies (150a, 150b) are fully deployed from distal end (136) of shaft assembly (130) as shown in FIG. 3B, the operator may press loop electrode assemblies (150a, 150b) and, more particularly, ring electrodes (170a, 170b) against the tissue that the operator wishes to ablate (or otherwise apply RF energy to). With the tissue adequately engaged by at least one first ring electrode (170a) and at least one second ring electrode (170b), the operator may then activate RF generator (102), with ring electrodes (170a, 170b) applying bipolar RF energy to the tissue against which loop electrode assemblies (150a, 150b) are pressed. In some scenarios, saline or other irrigation fluid may be expelled through shaft assembly (130) while RF energy is being applied to tissue via loop electrode assemblies (150a, 150b), thereby promoting electrical continuity. In addition, or in the alternative, suction may be applied via shaft assembly (130) and, more particularly, via open distal end (136), to evacuate smoke, vapor, etc. that is generated during the ablation procedure. In some alternative uses, shaft assembly (130) may be pressed into tissue such that distal end (136) penetrates the tissue to a certain depth. For instance, distal end (136) may be inserted through an incision that was formed using another instrument; or distal end (136) may be pressed with sufficient force to provide blunt dissection.

In some instances, an operator may wish to only partially deploy loop electrode assemblies (150a, 150b) from distal end (136) of shaft assembly (130). As shown in FIG. 3A, with slider (122) in a proximal-most position, loop electrode assemblies (150a, 150b) may be fully contained within shaft assembly (130). When slider (122) is partially advanced distally to an intermediate longitudinal position (not shown), loop electrode assemblies (150a, 150b) may partially extend distally from distal end (136) of shaft assembly (130) (not shown). The operator may nevertheless press the exposed ring electrodes (170a, 170b) against tissue when loop electrode assemblies (150a, 150b) are in a partially deployed state, and then activate at least the exposed ring electrodes (170a, 170b) to apply RF energy to tissue. If the operator chooses to fully deploy loop electrode assemblies (150a, 150b) in addition to or in lieu of applying RF energy to tissue while loop electrode assemblies (150a, 150b) are in the partially deployed state, the operator may continue to advance slider (122) to a distal position. With slider (122) in a distal position, loop electrode assemblies (150a, 150b) may be fully deployed and thereby define the generally bulbous shape shown in FIG. 3B. By way of example only, such partial and/or full deployment of loop electrode assemblies (150a, 150b) may be provided in accordance with at least some of the teachings of U.S. Pub. No. 2022/0054188, entitled "ENT Ablation Instrument with Electrode Loop," published February 24, 2022.

While not shown, instrument (100) may also include one or more position sensors that are operable to generate signals indicative of the position of distal end (136), or some other component of instrument (100), in three-dimensional space. Such a position sensor may be integrated directly into shaft assembly (130) or elsewhere into instrument (100) such as loop electrode assemblies (150a, 150b) as described below. In addition, or in the alternative, such a position sensor may be integrated into a guidewire or other component that is disposed in shaft assembly (130). Such a position sensor may take the form of one or more coils that generate signals in response to the presence of an alternating magnetic field. The position data generated by such position signals may be processed by a system (e.g., IGS navigation system (50) shown in FIG. 2 and described above) that provides a visual indication to the operator to show the operator where the distal end (136), or some other component of instrument (100), is located within the patient in real time. Such a visual indication may be provided as an overlay on one or more preoperatively obtained images (e.g., CT scans) of the patient's anatomy. Such position sensing and navigation capabilities may be provided in accordance with at least some of the teachings of the various references cited herein.

Additionally, in some versions, energy catheter (140) may further include one or more tissue sensors operable to sense a condition of the tissue (e.g., a nerve) being ablated by loop electrode assemblies (150a, 150b). Each such tissue sensor may communicate a signal to processor (52) indicating the sensed condition. In response to receiving the signal, processor (52) may then regulate (e.g., deactivate) the RF ablation energy being delivered to active ring electrodes (170a) from RF generator (102), and/or provide an indication to the operator informing of the sensed tissue condition. In some versions, such a tissue sensor may comprise a thermocouple operable to measure a temperature of the target tissue during ablation. In other versions, such a tissue sensor may comprise a pair of detection electrodes operable to deliver a low power RF signal to the target tissue to measure an electrical impedance of the tissue during ablation. In some such versions, such detection electrodes may be provided separately from ring electrodes (170a, 170b). In other such versions, one or more ring electrodes (170a, 170b) may be operable as both an ablation electrode and as a detection electrode. In either configuration, the low power RF signal may be delivered to the target tissue simultaneously or in rapidly alternating fashion with the high-power RF ablation energy delivered by ring electrodes (170a, 170b). While the target tissue remains substantially intact and unablated, the low power RF signal may pass freely through the tissue with a relatively low impedance. As ablation of the tissue progresses, the detection electrodes may detect a corresponding increase in impedance of the tissue, which is communicated to processor (52).

In some versions, each ring electrode (170a, 170b) may be activated independently relative to other ring electrodes (170a, 170b) to enable adjusting one or more ablation regions of tissue. As described above, such activation may be performed automatically by processor (52), such as in response to feedback received by processor (52) from the tissue sensors and/or position sensors to ensure proper ablation of the desired target tissue. In addition, or alternatively, such activation may be performed manually by the operator, such as based on images or other information displayed on display screen (56).

While loop electrode assemblies (150a, 150b) of the present version may be selectively advanced and retracted relative to shaft assembly (130) via slider (122), first loop electrode assembly (150a) and/or second loop electrode assembly (150b) may alternatively be longitudinally fixed relative to shaft assembly (130). In such versions, first loop electrode assembly (150a) and/or second loop electrode assembly (150b) may be selectively contained within, or exposed by, an outer sheath (not shown) that is slidably disposed relative to shaft assembly (130). For instance, shaft assembly (130) may slide longitudinally relative to such an outer sheath, or the outer sheath may slide longitudinally relative to shaft assembly (130), to selectively contain or expose first loop electrode assembly (150a) and/or second loop electrode assembly (150b). Regardless of how loop electrode assemblies (150a, 150b) are advanced, retracted, contained, or exposed, the degree of advancement, retraction, containment, or exposure may be selected and adjusted in a manner similar to that described above to thereby vary the degree of tissue contact.

### B. Alternative Energy Catheter Having Triple Loop Electrode Assemblies with Single Polarities

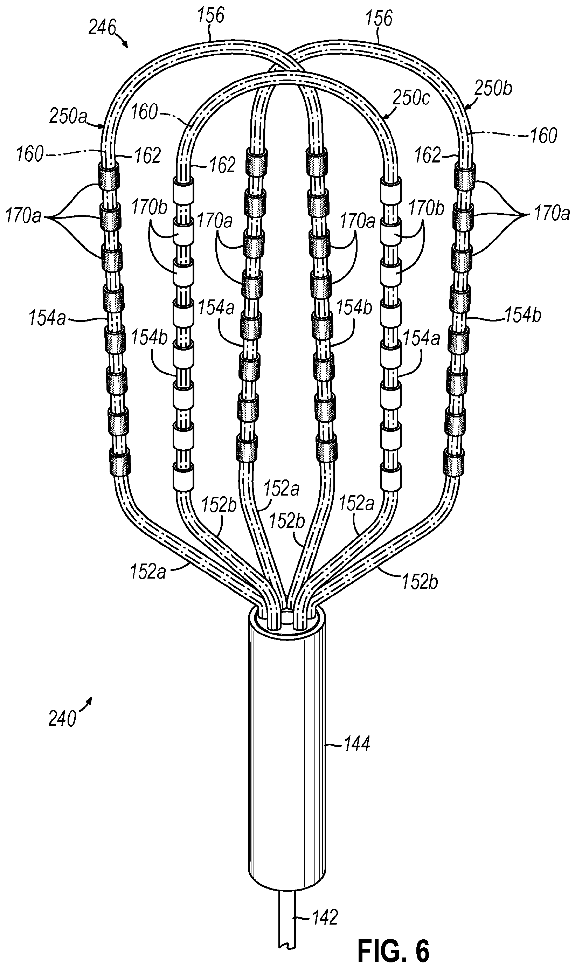

In some instances, it may be desirable to provide a catheter for use with instrument (100) having additional energy delivery capabilities as compared to energy catheter (140). FIG. 6 shows another exemplary energy catheter (240) having such capabilities. Energy catheter (240) is substantially similar to energy catheter (140) except as otherwise described herein.

Energy catheter (240) of the present example includes proximal body portion (142) and distal body portion (144), with an elongate end effector (246) having first, second, and third loop electrode assemblies (250a, 250b, 250c) extending distally from distal body portion (144). In this regard, and in embodiments not defined by the wording of the appended claims, loop electrode assemblies (250a, 250b, 250c) extend along respective planes that are each parallel to and positioned along the longitudinal axis of distal body portion (144) and/or the longitudinal axis of shaft assembly (130). In some other versions, and in accordance with the invention, the planes defined by loop electrode assemblies (250a, 250b, 250c) are parallel with, yet laterally offset from, the longitudinal axis of distal body portion (144) and/or the longitudinal axis of shaft assembly (130). Each loop electrode assembly (250a, 250b, 250c) includes first and second outwardly splayed segments (152a, 152b), first and second longitudinal segments (154a, 154b), and arcuate transverse segment (156).

In the example shown, and in accordance with embodiments of the invention, first and second loop electrode assemblies (250a, 250b) at least partially overlap each other. More particularly, first outwardly splayed segment (152a) of second loop electrode assembly (250b) connects to distal body portion (144) at a location between the locations at which outwardly splayed segments (152a, 152b) of first loop electrode assembly (250a) connect to distal body portion (144), and second outwardly splayed segment (152b) of first loop electrode assembly (250a) connects to distal body portion (144) at a location between the locations at which outwardly splayed segments (152a, 152b) of second loop electrode assembly (250b) connect to distal body portion (144). Third loop electrode assembly (250c) of the present version overlaps both of first and second loop electrode assemblies (250a, 250b). More particularly, outwardly splayed segments (152a, 152b) of third loop electrode assembly (250c) each connect to distal body portion (144) at locations between the locations at which first outwardly splayed segment (152a) of first loop electrode assembly (250a) and second outwardly splayed segment (152b) of second loop electrode assembly (250b) connect to distal body portion (144). In other versions, third loop electrode assembly (250c) may not overlap first and/or second loop electrode assemblies (250a, 250b), and/or first and second loop electrode assemblies (250a, 250b) may not overlap each other.

Similar to loop electrode assemblies (150a, 150b), each loop electrode assembly (250a, 250b, 250c) includes a flexible support wire (160), insulative sleeve (162), and a plurality of energy application elements in the form of first and second ring electrodes (170a, 170b). Loop electrode assembly (250a) has two parallel rows of first ring electrodes (170a), which are coaxially aligned with each other and are longitudinally spaced apart from each other along respective first and second longitudinal segments (154a, 154b) of loop electrode assembly (250a). Loop electrode assembly (250b) also has two parallel rows of first ring electrodes (170a), which are coaxially aligned with each other and are longitudinally spaced apart from each other along respective first and second longitudinal segments (154a, 154b) of loop electrode assembly (250b). Loop electrode assembly (250c) has two parallel rows of second ring electrodes (170b), which are coaxially aligned with each other and are longitudinally spaced apart from each other along respective first and second longitudinal segments (154a, 154b) of loop electrode assembly (250c).

With first and second loop electrode assemblies (250a, 250b) having respective sets of first ring electrodes (170a), and with third electrode assembly having sets of second ring electrodes (170b), first and second loop electrode assemblies (250a, 250b) are each configured to apply RF energy at a first polarity and third electrode assembly (250c) is configured to apply RF energy at a second polarity. Thus, first and second loop electrode assemblies (250a, 250b) may each cooperate with third loop electrode assembly (250c) to apply bipolar RF energy to tissue. In some versions, conductive cylinders (172) of first ring electrodes (170a) of first loop electrode assembly (250a) may be electrically coupled with RF generator (102) via a single conductive element (e.g., the corresponding support wire (160) of first loop electrode assembly (250a)), conductive cylinders (172) of first ring electrodes (170a) of second loop electrode assembly (250b) may be electrically coupled with RF generator (102) via a single conductive element (e.g., the corresponding support wire (160) of second loop electrode assembly (250b)), and conductive cylinders (172) of second ring electrodes (170b) of third loop electrode assembly (250c) may be electrically coupled with RF generator (102) via a single conductive element (e.g., the corresponding support wire (160) of third loop electrode assembly (250c)). Due to this arrangement of ring electrodes (170a, 170b) and the relationship of third loop electrode assembly (250c) with first and second loop electrode assemblies (250a, 250b), the polarities of ring electrodes (170a, 170b) may be divided by a plane defined between third loop electrode assembly (250c) and first and second loop electrode assemblies (250a, 250b).

### C. Alternative Energy Catheter Having Triple Loop Electrode Assemblies with Dual Polarities

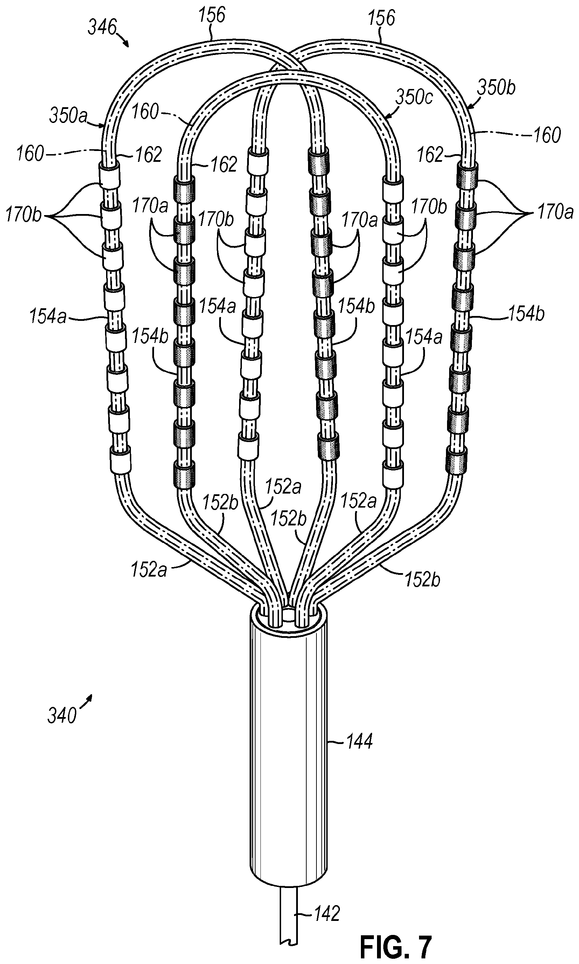

In some instances, it may be desirable to provide a catheter for use with instrument (100) having additional energy delivery capabilities as compared to energy catheter (140). FIG. 7 shows another exemplary energy catheter (340) having such capabilities. Energy catheter (340) is substantially similar to energy catheter (140) except as otherwise described herein.

Energy catheter (340) of the present example includes proximal body portion (142) and distal body portion (144), with an elongate end effector (346) having first, second, and third loop electrode assemblies (350a, 350b, 350c) extending distally from distal body portion (144). In this regard, and in embodiments not defined by the wording of the appended claims, loop electrode assemblies (350a, 350b, 350c) extend along respective planes that are each parallel to and positioned along the longitudinal axis of distal body portion (144) and/or the longitudinal axis of shaft assembly (130). In some other versions, and in accordance with the invention, the planes defined by loop electrode assemblies (350a, 350b, 350c) are parallel with, yet laterally offset from, the longitudinal axis of distal body portion (144) and/or the longitudinal axis of shaft assembly (130). Each loop electrode assembly (350a, 350b, 350c) includes first and second outwardly splayed segments (152a, 152b), first and second longitudinal segments (154a, 154b), and arcuate transverse segment (156).

In the example shown, and in accordance with embodiments of the invention, first and second loop electrode assemblies (350a, 350b) at least partially overlap each other. More particularly, first outwardly splayed segment (152a) of second loop electrode assembly (350b) connects to distal body portion (144) at a location between the locations at which outwardly splayed segments (152a, 152b) of first loop electrode assembly (350a) connect to distal body portion (144), and second outwardly splayed segment (152b) of first loop electrode assembly (350a) connects to distal body portion (144) at a location between the locations at which outwardly splayed segments (152a, 152b) of second loop electrode assembly (350b) connect to distal body portion (144). Third loop electrode assembly (350c) of the present version overlaps both of first or second loop electrode assemblies (350a, 350b). More particularly, outwardly splayed segments (152a, 152b) of third loop electrode assembly (350c) each connect to distal body portion (144) at locations between the locations at which first outwardly splayed segment (152a) of first loop electrode assembly (350a) and second outwardly splayed segment (152b) of second loop electrode assembly (350b) connect to distal body portion (144). In other versions, third loop electrode assembly (350c) may not overlap first and/or second loop electrode assemblies (350a, 350b), and/or first and second loop electrode assemblies (350a, 350b) may not overlap each other.

Similar to loop electrode assemblies (150a, 150b), each loop electrode assembly (350a, 350b, 350c) includes flexible support wire (160), insulative sleeve (162), and a plurality of energy application elements in the form of first and second ring electrodes (170a, 170b). Loop electrode assembly (350a) has a row of first ring electrodes (170a) and a row of second ring electrodes (170a). First ring electrodes (170a) of loop electrode assembly (350a) are coaxially aligned with each other and are longitudinally spaced apart from each other along a second longitudinal segment (154b) of loop electrode assembly (350a); while second ring electrodes (170b) of loop electrode assembly (350a) are coaxially aligned with each other and are longitudinally spaced apart from each other along a first longitudinal segment (154a) of loop electrode assembly (350a). First ring electrodes (170a) of loop electrode assembly (350a) are operable to apply RF energy at a first polarity while second ring electrodes (170b) of loop electrode assembly (350a) are operable to apply RF energy at a second polarity. Loop electrode assembly (350a) is thus operable, by itself, to apply bipolar RF energy to tissue.

Similarly, loop electrode assembly (350b) has a row of first ring electrodes (170a) and a row of second ring electrodes (170a). First ring electrodes (170a) of loop electrode assembly (350b) are coaxially aligned with each other and are longitudinally spaced apart from each other along a second longitudinal segment (154b) of loop electrode assembly (350b); while second ring electrodes (170b) of loop electrode assembly (350b) are coaxially aligned with each other and are longitudinally spaced apart from each other along a first longitudinal segment (154a) of loop electrode assembly (350b). First ring electrodes (170a) of loop electrode assembly (350b) are operable to apply RF energy at a first polarity while second ring electrodes (170b) of loop electrode assembly (350b) are operable to apply RF energy at a second polarity. Loop electrode assembly (350b) is thus operable, by itself, to apply bipolar RF energy to tissue.

Similarly, loop electrode assembly (350c) has a row of first ring electrodes (170a) and a row of second ring electrodes (170a). First ring electrodes (170a) of loop electrode assembly (350c) are coaxially aligned with each other and are longitudinally spaced apart from each other along a second longitudinal segment (154b) of loop electrode assembly (350c); while second ring electrodes (170b) of loop electrode assembly (350c) are coaxially aligned with each other and are longitudinally spaced apart from each other along a first longitudinal segment (154a) of loop electrode assembly (350c). First ring electrodes (170a) of loop electrode assembly (350c) are operable to apply RF energy at a first polarity while second ring electrodes (170b) of loop electrode assembly (350c) are operable to apply RF energy at a second polarity. Loop electrode assembly (350c) is thus operable, by itself, to apply bipolar RF energy to tissue.

In some versions, conductive cylinders (172) of first ring electrodes (170a) of each second longitudinal segment (154b) may be electrically coupled with RF generator (102) via a respective first conductive element (e.g., received within the passageway of the corresponding insulative sleeve (162) along the respective second longitudinal segment (154b) and second outwardly splayed segment (152b)), and conductive cylinders (172) of second ring electrodes (170b) of each first longitudinal segment (154a) may be electrically coupled with RF generator (102) via a respective second conductive element (e.g., received within the passageway of the corresponding insulative sleeve (162) along the respective first longitudinal segment (154a) and first outwardly splayed segment (152a)).

While each loop electrode assembly (350, 350b, 350c) may independently apply bipolar RF energy to tissue, loop electrode assemblies (350a, 350b, 350c) may also cooperate with each other to apply bipolar RF energy to tissue. Due to the arrangement of ring electrodes (170a, 170b) and the relationship of third loop electrode assembly (350c) with first and second loop electrode assemblies (350a, 350b), the polarities of ring electrodes (170a, 170b) may be arranged in a single-alternating sequence in a first circumferential direction between first longitudinal segment (154a) of first loop electrode assembly (350a) and second longitudinal segment (154b) of second loop electrode assembly (350b), and may be arranged in a double-alternating sequence in a second circumferential direction between first longitudinal segment (154a) of first loop electrode assembly (350a) and second longitudinal segment (154b) of second loop electrode assembly (350b).

### D. Alternative Energy Catheter Having Triple Loop Electrode Assemblies with Dual Polarities and Position Sensors

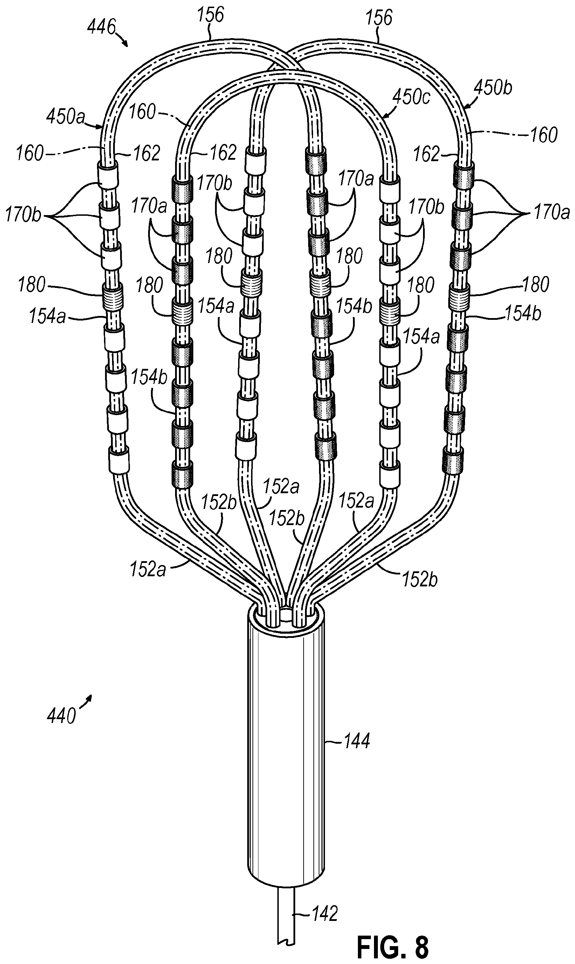

In some instances, it may be desirable to provide a catheter for use with instrument (100) having additional energy delivery capabilities and/or additional position sensing capabilities as compared to energy catheter (140). FIG. 8 shows another exemplary energy catheter (440) having such capabilities. Energy catheter (440) is substantially similar to energy catheter (140) except as otherwise described herein.

Energy catheter (440) of the present example includes proximal body portion (142) and distal body portion (144), with an elongate end effector (446) having first, second, and third loop electrode assemblies (450a, 450b, 450c) extending distally from distal body portion (144). In this regard, and in embodiments not defined by the wording of the appended claims, loop electrode assemblies (450a, 450b, 450c) extend along respective planes that are each parallel to and positioned along the longitudinal axis of distal body portion (144) and/or the longitudinal axis of shaft assembly (130). In some other versions, and in accordance with the invention, the planes defined by loop electrode assemblies (450a, 450b, 450c) are parallel with, yet laterally offset from, the longitudinal axis of distal body portion (144) and/or the longitudinal axis of shaft assembly (130). Each loop electrode assembly (450a, 450b, 450c) includes first and second outwardly splayed segments (152a, 152b), first and second longitudinal segments (154a, 154b), and arcuate transverse segment (156).

Similar to loop electrode assemblies (150a, 150b), each loop electrode assembly (450a, 450b, 450c) includes flexible support wire (160), insulative sleeve (162), and a plurality of energy application elements in the form of first and second ring electrodes (170a, 170b). First ring electrodes (170a) are positioned along second longitudinal segments (154b) of each loop electrode assembly (450a, 450b, 450c), and second ring electrodes (170b) are positioned along first longitudinal segments (154a) of each loop electrode assembly (450a, 450b, 450c), such that second longitudinal segments (154b) are each configured to apply RF energy at a first polarity and first longitudinal segments (154a) are each configured to apply RF energy at a second polarity. Loop electrode assemblies (450a, 450b, 450c) are thus configured and operable similar to loop electrode assembly (350a, 350b, 350c) in this regard.

In the example shown, each loop electrode assembly (450a, 450b, 450c) further includes at least one position sensor (180) positioned on each of the respective first and second longitudinal segments (154a, 154b) (e.g., coaxially with and/or between the respective ring electrodes (170a, 170b)). Each position sensor (180) is operable to generate signals indicative of the position of the respective loop electrode assembly (450a, 450b, 450c) in three-dimensional space. Position sensors (180) may further indicate the geometric shape of the respective loop electrode assembly (450a, 450b, 450c) and/or the pattern of RF energy applied by the corresponding ring electrodes (170a, 170b) in real time, including when loop electrode assemblies (450a, 450b, 450c) are deformed as loop electrode assemblies (450a, 450b, 450c) are being pressed against tissue.

Each position sensor (180) may take the form of one or more coils that generate signals in response to the presence of an alternating magnetic field. The position data generated by such position signals may be processed by a system (e.g., IGS navigation system (50)) that provides a visual indication to the operator to show the operator where the respective loop electrode assembly (450a, 450b, 450c) is located within the patient in real time. Such a visual indication may be provided as an overlay on one or more preoperatively obtained images (e.g., CT scans) of the patient's anatomy. Such position sensing and navigation capabilities may be provided in accordance with at least some of the teachings of the various references cited herein. In some versions, position sensors (180) may each be operatively coupled to processor (52) via one or more wires (not shown) received within the passageway of the corresponding insulative sleeve (162) for transmitting position signals thereto.

### E. Alternative Energy Catheter Having Triple Loop Electrode Assemblies with Dual Polarities

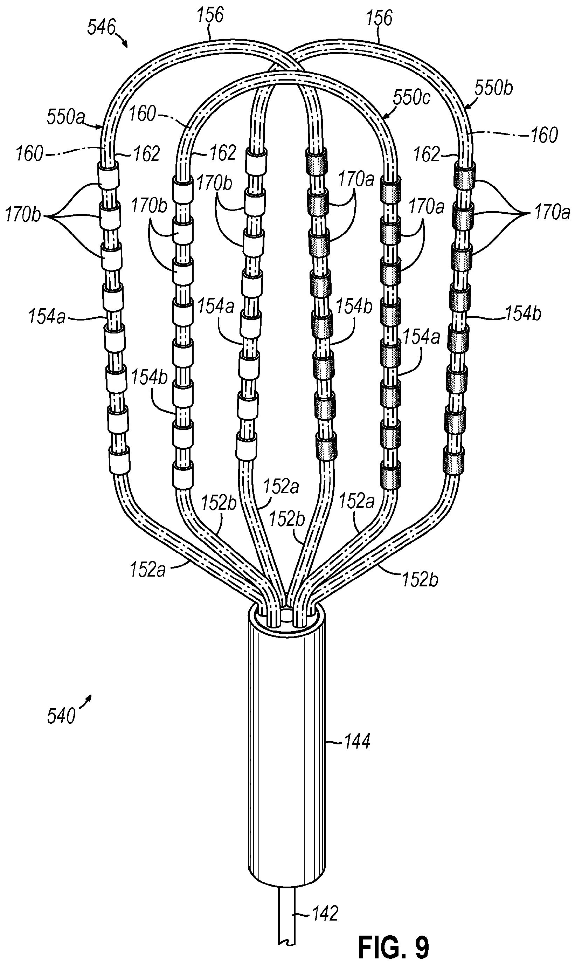

In some instances, it may be desirable to provide a catheter for use with instrument (100) having additional energy delivery capabilities as compared to energy catheter (140). FIG. 9 shows another exemplary energy catheter (540) having such capabilities. Energy catheter (540) is substantially similar to energy catheter (140) except as otherwise described herein.

Energy catheter (540) of the present example includes proximal body portion (142) and distal body portion (144), with an elongate end effector (546) having first, second, and third loop electrode assemblies (550a, 550b, 550c) extending distally from distal body portion (144). In this regard, and in embodiments not defined by the wording of the appended claims, loop electrode assemblies (550a, 550b, 550c) extend along respective planes that are each parallel to and positioned along the longitudinal axis of distal body portion (144) and/or the longitudinal axis of shaft assembly (130). In some other versions, and in accordance with the invention, the planes defined by loop electrode assemblies (550a, 550b, 550c) are parallel with, yet laterally offset from, the longitudinal axis of distal body portion (144) and/or the longitudinal axis of shaft assembly (130). Each loop electrode assembly (550a, 550b, 550c) includes first and second outwardly splayed segments (152a, 152b), first and second longitudinal segments (154a, 154b), and arcuate transverse segment (156).

In the example shown, and in accordance with embodiments of the invention, first and second loop electrode assemblies (550a, 550b) at least partially overlap each other. More particularly, first outwardly splayed segment (152a) of second loop electrode assembly (550b) connects to distal body portion (144) at a location between the locations at which outwardly splayed segments (152a, 152b) of first loop electrode assembly (550a) connect to distal body portion (144), and second outwardly splayed segment (152b) of first loop electrode assembly (550a) connects to distal body portion (144) at a location between the locations at which outwardly splayed segments (152a, 152b) of second loop electrode assembly (550b) connect to distal body portion (144). Third loop electrode assembly (550c) of the present version overlaps both of first and second loop electrode assemblies (550a, 550b). More particularly, outwardly splayed segments (152a, 152b) of third loop electrode assembly (550c) each connect to distal body portion (144) at locations between the locations at which first outwardly splayed segment (152a) of first loop electrode assembly (550a) and second outwardly splayed segment (152b) of second loop electrode assembly (550b) connect to distal body portion (144). In other versions, third loop electrode assembly (550c) may not overlap first and/or second loop electrode assemblies (550a, 550b), and/or first and second loop electrode assemblies (550a, 550b) may not overlap each other.

Similar to loop electrode assemblies (150a, 150b), each loop electrode assembly (550a, 550b, 550c) includes flexible support wire (160), insulative sleeve (162), and a plurality of energy application elements in the form of first and second ring electrodes (170a, 170b). Loop electrode assembly (550a) has a row of first ring electrodes (170a) and a row of second ring electrodes (170a). First ring electrodes (170a) of loop electrode assembly (550a) are coaxially aligned with each other and are longitudinally spaced apart from each other along a second longitudinal segment (154b) of loop electrode assembly (550a); while second ring electrodes (170b) of loop electrode assembly (550a) are coaxially aligned with each other and are longitudinally spaced apart from each other along a first longitudinal segment (154a) of loop electrode assembly (550a). First ring electrodes (170a) of loop electrode assembly (550a) are operable to apply RF energy at a first polarity while second ring electrodes (170b) of loop electrode assembly (550a) are operable to apply RF energy at a second polarity. Loop electrode assembly (550a) is thus operable, by itself, to apply bipolar RF energy to tissue.

Similarly, loop electrode assembly (550b) has a row of first ring electrodes (170a) and a row of second ring electrodes (170a). First ring electrodes (170a) of loop electrode assembly (550b) are coaxially aligned with each other and are longitudinally spaced apart from each other along a second longitudinal segment (154b) of loop electrode assembly (550b); while second ring electrodes (170b) of loop electrode assembly (550b) are coaxially aligned with each other and are longitudinally spaced apart from each other along a first longitudinal segment (154a) of loop electrode assembly (550b). First ring electrodes (170a) of loop electrode assembly (550b) are operable to apply RF energy at a first polarity while second ring electrodes (170b) of loop electrode assembly (550b) are operable to apply RF energy at a second polarity. Loop electrode assembly (550b) is thus operable, by itself, to apply bipolar RF energy to tissue.

Similarly, loop electrode assembly (550c) has a row of first ring electrodes (170a) and a row of second ring electrodes (170a). First ring electrodes (170a) of loop electrode assembly (550c) are coaxially aligned with each other and are longitudinally spaced apart from each other along a first longitudinal segment (154a) of loop electrode assembly (550c); while second ring electrodes (170b) of loop electrode assembly (550c) are coaxially aligned with each other and are longitudinally spaced apart from each other along a second longitudinal segment (154b) of loop electrode assembly (550c). First ring electrodes (170a) of loop electrode assembly (550c) are operable to apply RF energy at a first polarity while second ring electrodes (170b) of loop electrode assembly (550c) are operable to apply RF energy at a second polarity. Loop electrode assembly (550c) is thus operable, by itself, to apply bipolar RF energy to tissue.

While each loop electrode assembly (550, 550b, 550c) may independently apply bipolar RF energy to tissue, loop electrode assemblies (550a, 550b, 550c) may also cooperate with each other to apply bipolar RF energy to tissue. End effector (546) is thus configured and operable similar to end effector (346) described above. However, due to the arrangement of ring electrodes (170a, 170b) and the overlapping relationship of first loop electrode assembly (550a) with second loop electrode assembly (550b) in end effector (346), the polarities of ring electrodes (170a, 170b) in end effector (546) are distributed on opposing lateral sides relative to the longitudinal axis of distal body portion (144) and/or the longitudinal axis of shaft assembly (130). In other words, in end effector (546) first electrodes (170a) are ganged together on a first lateral side of the longitudinal axis to apply the first polarity on the first lateral side of the longitudinal axis; while electrodes (170b) are ganged together on the second lateral side of the longitudinal axis to apply the second polarity on the second lateral side of the longitudinal axis.

### F. Alternative Energy Catheter Having Triple Loop Electrode Assemblies with Dual Polarities and Position Sensors

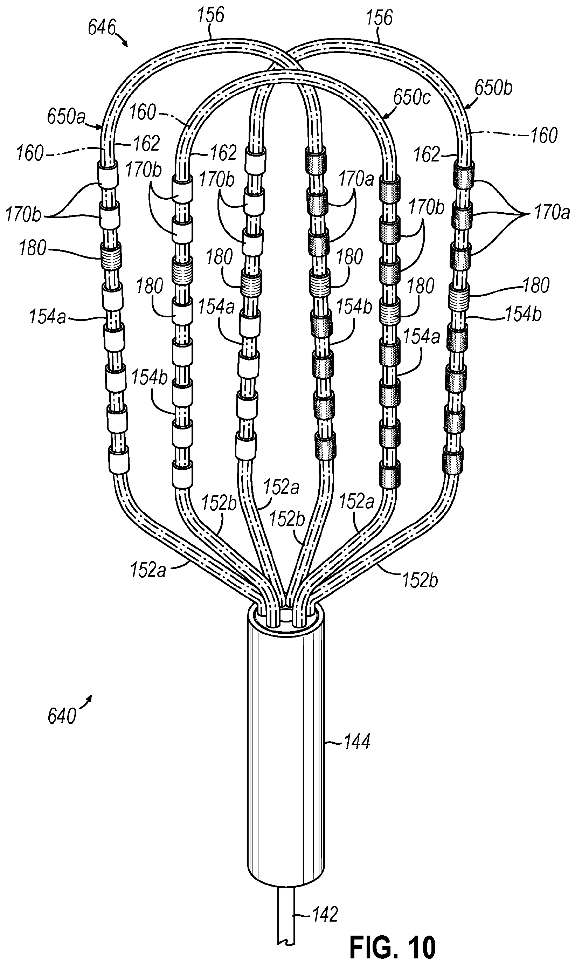

In some instances, it may be desirable to provide a catheter for use with instrument (100) having additional energy delivery capabilities and/or additional position sensing capabilities as compared to energy catheter (140). FIG. 10 shows another exemplary energy catheter (640) having such capabilities. Energy catheter (640) is substantially similar to energy catheter (140) except as otherwise described herein.

Energy catheter (640) of the present example includes proximal body portion (142) and distal body portion (144), with an elongate end effector (646) having first, second, and third loop electrode assemblies (650a, 650b, 650c) extending distally from distal body portion (144). In this regard, and in embodiments not defined by the wording of the appended claims, loop electrode assemblies (650a, 650b, 650c) extend along respective planes that are each parallel to and positioned along the longitudinal axis of distal body portion (144) and/or the longitudinal axis of shaft assembly (130). In some other versions, and in accordance with the invention, the planes defined by loop electrode assemblies (650a, 650b, 650c) are parallel with, yet laterally offset from, the longitudinal axis of distal body portion (144) and/or the longitudinal axis of shaft assembly (130). Each loop electrode assembly (650a, 650b, 650c) includes first and second outwardly splayed segments (152a, 152b), first and second longitudinal segments (154a, 154b), and arcuate transverse segment (156).

Similar to loop electrode assemblies (150a, 150b), loop electrode assemblies (650a, 650b, 650c) each include flexible support wire (160), insulative sleeve (162), and a plurality of energy application elements in the form of first and second ring electrodes (170a, 170b). First ring electrodes (170a) are positioned along second longitudinal segments (154b) of first and second loop electrode assemblies (650a, 650b) and first longitudinal segment (154a) of third loop electrode assembly (650c), and second ring electrodes (170b) are positioned along first longitudinal segments (154a) of first and second loop electrode assemblies (650a, 650b) and second longitudinal segment (154b) of third loop electrode assembly (650c), such that second longitudinal segments (154b) of first and second loop electrode assemblies (650a, 650b) and first longitudinal segment (154a) of third loop electrode assembly (650c) are each configured to apply RF energy at a first polarity and first longitudinal segments (154a) of first and second loop electrode assemblies (650a, 650b) and second longitudinal segment (154b) of third loop electrode assembly (650c) are each configured to apply RF energy at a second polarity. Loop electrode assemblies (650a, 650b, 650c) are thus configured and operable similar to loop electrode assembly (550a, 550b, 550c) in this regard.

In the example shown, each loop electrode assembly (650a, 650b, 650c) further includes at least one position sensor (180) positioned on each of the respective first and second longitudinal segments (154a, 154b) (e.g., coaxially with and/or between the respective ring electrodes (170a, 170b)). Each position sensor (180) is operable to generate signals indicative of the position of the respective loop electrode assembly (650a, 650b, 650c) in three-dimensional space. Position sensors (180) may further indicate the geometric shape of the respective loop electrode assembly (650a, 650b, 650c) and/or the pattern of RF energy applied by the corresponding ring electrodes (170a, 170b) in real time, including when loop electrode assemblies (650a, 650b, 650c) are deformed as loop electrode assemblies (650a, 650b, 650c) are being pressed against tissue. Position sensors (180) in end effector (646) may thus be configured and operable similar to position sensors in end effector (446).

### G. Alternative End Effector Having Single Loop Electrode Assembly with Dual Polarities

In some instances, it may be desirable to provide an elongate end effector for use with instrument (100) having different energy delivery capabilities and/or a flatter profile as compared to end effector (146). FIG. 11 shows another exemplary end effector (746) having such capabilities. End effector (746) is substantially similar to end effector (146) except as otherwise described herein.

End effector (746) of the present example has a loop electrode assembly (750) that may extend distally from a distal body portion of a corresponding energy catheter (not shown) along a plane that is parallel to and/or aligned with the longitudinal axis of such a distal body portion (144) and/or the longitudinal axis of shaft assembly (130). Loop electrode assembly (750) includes first and second proximal longitudinal segments (751a, 751b), first and second outwardly splayed segments (752a, 752b) coupled to distal ends of respective proximal longitudinal segments (751a, 751b), first and second distal longitudinal segments (754a, 754b) extending distally from distal ends of respective outwardly splayed segments (752a, 752b), and an arcuate transverse segment (756) extending between distal ends of distal longitudinal segments (754a, 754b). In the present example, loop electrode assembly (750) defines a generally bulbous shape. While loop electrode assembly (750) is generally symmetric, loop electrode assembly (750) may be asymmetric if desired.

Similar to loop electrode assemblies (150a, 150b), loop electrode assembly (750) may include a flexible support wire (not shown) resiliently biased to define the generally bulbous shape of loop electrode assembly (750), an insulative sleeve (762), and a plurality of energy application elements in the form of first and second ring electrodes (170a, 170b). First ring electrodes (170a) are positioned along second distal longitudinal segment (754b) of loop electrode assembly (750), and second ring electrodes (170b) are positioned along first distal longitudinal segment (754a) of loop electrode assemblies (750), such that second distal longitudinal segment (754b) is configured to apply RF energy at a first polarity and first distal longitudinal segment (754a) is configured to apply RF energy at a second polarity. Due to this arrangement of ring electrodes (170a, 170b), the polarities of ring electrodes (170a, 170b) are distributed on opposing lateral sides relative to the longitudinal axis of shaft assembly (130). End effector (746) may be used to apply bipolar RF energy to tissue as described herein (e.g., to provide ablation, electroporation, etc.).

### H. Alternative End Effector Having Single Loop Electrode Assembly and Straight Electrode Assembly with Single Polarities

In some instances, it may be desirable to provide an end elongate effector for use with instrument (100) having different energy delivery capabilities and/or a flatter profile as compared to end effector (146). FIG. 12 shows another exemplary end effector (846) having such capabilities. End effector (846) is substantially similar to end effector (146) except as otherwise described herein.

End effector (846) of the present example has a loop electrode assembly (850) that may extend distally from a distal body portion of a corresponding energy catheter (not shown) along a plane that is parallel to and/or aligned with the longitudinal axis of such a distal body portion and/or the longitudinal axis of shaft assembly (130). Loop electrode assembly (850) includes first and second proximal longitudinal segments (751a, 751b), first and second outwardly splayed segments (752a, 752b), first and second distal longitudinal segments (754a, 754b), and arcuate transverse segment (756). In the example shown, end effector (146) also has a straight electrode assembly (858) that may extend distally from the distal body portion of the corresponding energy catheter coaxially with the distal body portion and/or shaft assembly (130). As shown, straight electrode assembly (858) is nested within the perimeter defined by loop electrode assembly (850) and is coplanar with loop electrode assembly (850). A proximal portion of straight electrode assembly (858) is laterally interposed between first and second proximal longitudinal segments (751a, 751b) of loop electrode assembly (850).

Similar to loop electrode assemblies (150a, 150b), loop electrode assembly (850) may include a flexible support wire (not shown) resiliently biased to define the generally bulbous shape of loop electrode assembly (850), insulative sleeve (762), and a plurality of energy application elements in the form of first ring electrodes (170a). First ring electrodes (170a) are positioned along first and second distal longitudinal segments (754a, 754b) of loop electrode assembly (850), such that first and second distal longitudinal segments (754a, 754b) are each configured to apply RF energy at a first polarity. Straight electrode assembly (858) may likewise include a flexible support wire (not shown) resiliently biased to define the generally elongate shape of straight electrode assembly (858), an insulative sleeve (862), and a plurality of energy application elements in the form of second ring electrodes (170b). In this manner, straight electrode assembly (858) may be configured to apply RF energy at a second polarity. Thus, loop electrode assembly (850) and straight electrode assembly (858) may cooperate with each other to apply bipolar RF energy to tissue between straight electrode assembly (858) and first distal longitudinal segment (754a) and/or between straight electrode assembly (858) and second distal longitudinal segment (754b).

### I. Alternative End Effector Having Double Loop Electrode Assemblies with Single Polarities

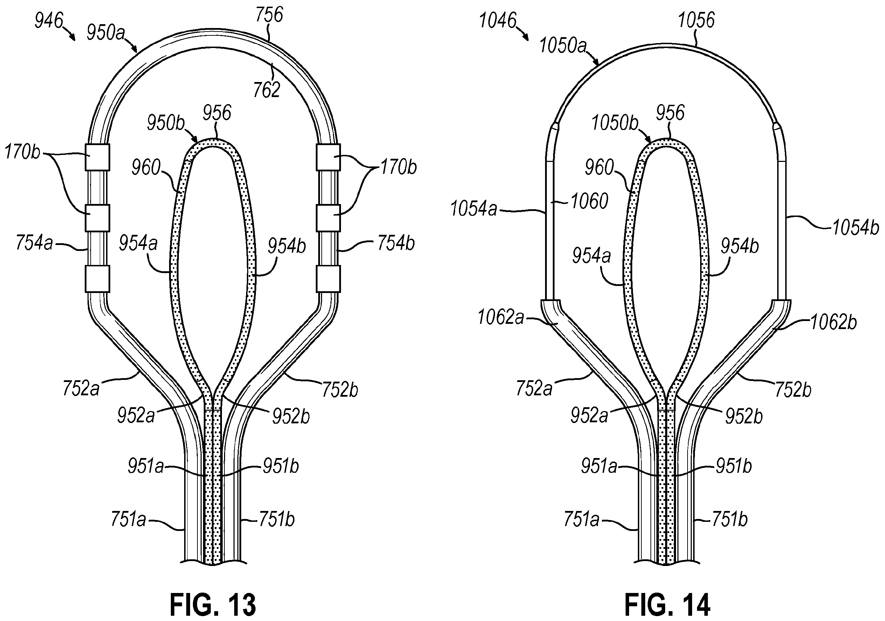

In some instances, it may be desirable to provide an elongate end effector for use with instrument (100) having different energy delivery capabilities and/or a flatter profile as compared to end effector (146). FIG. 13 shows another exemplary end effector (946) having such capabilities. End effector (946) is substantially similar to end effector (146) except as otherwise described herein.

End effector (946) of the present example has first and second loop electrode assemblies (950a, 950b) that may each extend distally from a distal body portion of a corresponding energy catheter (not shown) along a plane that is parallel to and/or aligned with the longitudinal axis of such a distal body portion and/or the longitudinal axis of shaft assembly (130). First loop electrode assembly (950a) includes first and second proximal longitudinal segments (751a, 751b), first and second outwardly splayed segments (752a, 752b), first and second distal longitudinal segments (754a, 754b), and arcuate transverse segment (756). Second loop electrode assembly (950b) includes first and second straight longitudinal segments (951a, 951b), first and second outwardly splayed segments (952a, 952b) coupled to distal ends of respective straight longitudinal segments (951a, 951b), first and second arcuate longitudinal segments (954a, 954b) extending distally from distal ends of respective outwardly splayed segments (952a, 952b), and an arcuate transverse segment (956) extending between distal ends of arcuate longitudinal segments (954a, 954b). In the present example, first loop electrode assembly (950a) defines a generally bulbous shape, and second loop electrode assembly (950b) defines a generally elliptical shape. As shown, second loop electrode assembly (950b) is nested within the perimeter defined by first loop electrode assembly (950a) and is coplanar with first loop electrode assembly (950a). First and second straight longitudinal segments (951a, 951b) of second loop electrode assembly (950b) are laterally interposed between first and second proximal longitudinal segments (751a, 751b) of first loop electrode assembly (950a).

Similar to loop electrode assemblies (150a, 150b), first loop electrode assembly (950a) may include a flexible support wire (not shown) resiliently biased to define the generally bulbous shape of first loop electrode assembly (950a), insulative sleeve (762), and a plurality of energy application elements in the form of second ring electrodes (170b). Second ring electrodes (170b) are positioned along first and second distal longitudinal segments (754a, 754b) of first loop electrode assembly (950a), such that first and second distal longitudinal segments (754a, 754b) are each configured to apply RF energy at a second polarity. In the present example, second loop electrode assembly (950b) includes an energy application element in the form of a flexible support wire (960) resiliently biased to define the generally elliptical shape of second loop electrode assembly (950b) and formed of an electrically conductive material, such as nitinol. In some versions, second loop electrode assembly (950b) may include an insulative sleeve (not shown) partially covering flexible support wire (960) with various predetermined electrode portions of flexible support (960) being left exposed. In any event, flexible support wire (960) is coupled with a corresponding one or more wire(s), trace(s), and/or other conductive element(s) that electrically couple flexible support wire (960) with RF generator (102). In this manner, second loop electrode assembly (950b) may be configured to apply RF energy at a first polarity. Thus, first and second loop electrode assemblies (950a, 950b) may cooperate with each other to apply bipolar RF energy to tissue between first and second loop electrode assemblies (950a, 950b).

### J. Alternative End Effector Having Double Loop Electrode Assemblies with Single Polarities

In some instances, it may be desirable to provide an elongate end effector for use with instrument (100) having different energy delivery capabilities and/or a flatter profile as compared to end effector (146). FIG. 14 shows another exemplary end effector (1046) having such capabilities. End effector (1046) is substantially similar to end effector (146) except as otherwise described herein.

End effector (1046) of the present example has first and second loop electrode assemblies (1050a, 1050b) that may each extend distally from a distal body portion of a corresponding energy catheter (not shown) along a plane that is parallel to and/or aligned with the longitudinal axis of such a distal body portion and/or the longitudinal axis of shaft assembly (130). First loop electrode assembly (1050a) includes first and second proximal longitudinal segments (751a, 751b), first and second outwardly splayed segments (752a, 752b), first and second distal longitudinal segments (1054a, 1054b) extending distally from distal ends of respective outwardly splayed segments (752a, 752b), and an arcuate transverse segment (1056) extending between distal ends of distal longitudinal segments (1054a, 1054b). Second loop electrode assembly (1050b) includes first and second straight longitudinal segments (951a, 951b), first and second outwardly splayed segments (952a, 952b), first and second arcuate longitudinal segments (954a, 954b), and an arcuate transverse segment (956). In the present example, first loop electrode assembly (1050a) defines a generally bulbous shape, and second loop electrode assembly (1050b) defines a generally elliptical shape. As shown, second loop electrode assembly (1050b) is nested within the perimeter defined by first loop electrode assembly (1050a) and is coplanar therewith, and first and second straight longitudinal segments (951a, 951b) of second loop electrode assembly (1050b) are interposed between first and second proximal longitudinal segments (751a, 751b) of first loop electrode assembly (1050a).

In the present example, first loop electrode assembly (1050a) includes an energy application element in the form of a flexible support wire (1060) resiliently biased to define the generally bulbous shape of first loop electrode assembly (1050a) and formed of an electrically conductive material, such as nitinol. First loop electrode assembly (1050a) also includes a pair of insulative sleeves (1062a, 1062b) extending along respective proximal longitudinal segments (751a, 751b) and outwardly splayed segments (752a, 752b) over flexible support wire (1060) to prevent short circuiting between loop electrode assemblies (1050a, 1050b). In the example shown, flexible support wire (1060) is relatively thick along distal longitudinal segments (1054a, 1054b) and relatively thin along arcuate transverse segment (1056). Flexible support wire (1060) may alternatively have a constant thickness along the entirety of the length of flexible support wire (1060).

Flexible support wire (1060) is coupled with a corresponding one or more wire(s), trace(s), and/or other conductive element(s) that electrically couple flexible support wire (1060) with RF generator (102). In this manner, first and second distal longitudinal segments (1054a, 1054b) and arcuate transverse segment (1056) may each be configured to apply RF energy at one of a first or second polarity, such as a second polarity. In the present example, second loop electrode assembly (1050b) includes an energy application element in the form of flexible support wire (960) resiliently biased to define the generally elliptical shape of second loop electrode assembly (1050b). In some versions, second loop electrode assembly (1050b) may include an insulative sleeve (not shown) partially covering flexible support wire (960) with various predetermined electrode portions of flexible support (960) being left exposed. In any event, flexible support wire (960) is coupled with a corresponding one or more wire(s), trace(s), and/or other conductive element(s) that electrically couple flexible support wire (960) with RF generator (102). In this manner, second loop electrode assembly (1050b) may be configured to apply RF energy at the other of a first or second polarity, such as a first polarity. Thus, first and second loop electrode assemblies (1050a, 1050b) may cooperate with each other to apply bipolar RF energy to tissue between first and second loop electrode assemblies (1050a, 1050b).

### K. Alternative End Effector Having Triple Loop Electrode Assemblies with Dual Polarities and Removable Energy Application Elements

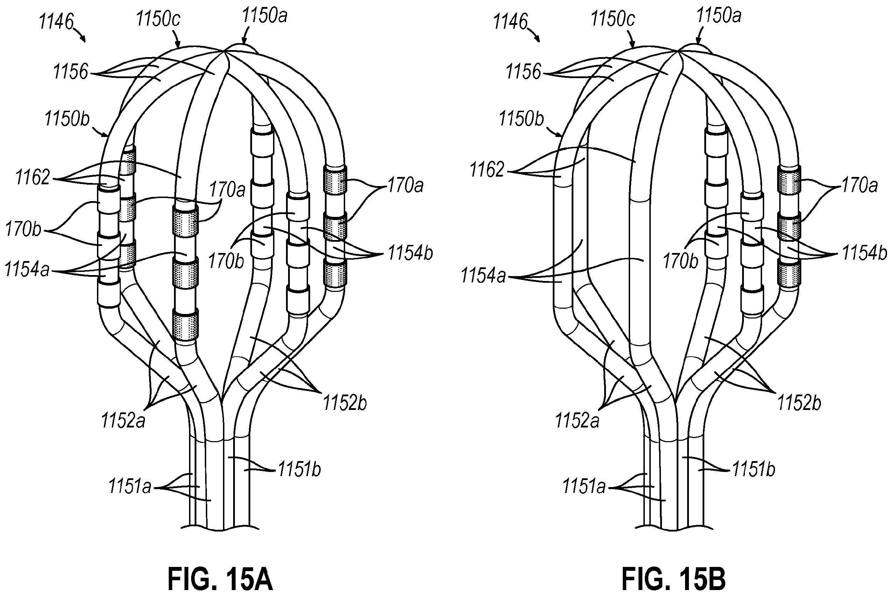

In some instances, it may be desirable to provide an elongate end effector for use with instrument (100) having additional energy delivery capabilities and/or additional energy delivery adjustment capabilities as compared to end effector (146). FIGS. 15A-15B show another exemplary end effector (1146) having such capabilities. End effector (1146) is substantially similar to end effector (146) except as otherwise described herein.

End effector (1146) of the present example has first, second, and third loop electrode assemblies (1150a, 1150b, 1150c) that may extend distally from a distal body portion of a corresponding energy catheter (not shown) along respective planes that may each be parallel to and/or aligned with the longitudinal axis of such a distal body portion and/or the longitudinal axis of shaft assembly (130). Each loop electrode assembly (1150a, 1150b, 1150c) includes first and second proximal longitudinal segments (1151a, 1151b), first and second outwardly splayed segments (1152a, 1152b) coupled to distal ends of respective proximal longitudinal segments (1151a, 1151b), first and second distal longitudinal segments (1154a, 1154b) extending distally from distal ends of respective outwardly splayed segments (1152a, 1152b), and an arcuate transverse segment (1156) extending between distal ends of distal longitudinal segments (1154a, 1154b). In the example shown, first, second, and third loop electrode assemblies (1150a, 1150b, 1150c) each define a generally bulbous shape and intersect each other at apexes of the respective arcuate transverse segments (1156).

Similar to loop electrode assemblies (150a, 150b), each loop electrode assembly (1150a, 1150b, 1150c) includes a flexible support wire (not shown), an insulative sleeve (1162), and a plurality of energy application elements in the form of first and second ring electrodes (170a, 170b). As shown in FIG. 15A, first ring electrodes (170a) are selectively positioned along first distal longitudinal segments (1154a) of first and third loop electrode assemblies (1150a, 1150c) and second distal longitudinal segment (1154b) of second loop electrode assembly (1150b). First distal longitudinal segments (1154a) of first and third loop electrode assemblies (1150a, 1150c) and second distal longitudinal segment (1154b) of second loop electrode assembly (1150b) are each thus configured to apply RF energy at a first polarity. Second ring electrodes (170b) are selectively positioned along second distal longitudinal segments (1154b) of first and third loop electrode assemblies (1150a, 1150c) and first distal longitudinal segment (1154a) of second loop electrode assembly (1150b). Second distal longitudinal segments (1154b) of first and third loop electrode assemblies (1150a, 1150c) and first distal longitudinal segment (1154a) of second loop electrode assembly (1150b) are thus each configured to apply RF energy at a second polarity.

In the example shown, at least a portion of first and/or second ring electrodes (170a, 170b) are selectively removable from the respective longitudinal segment(s) (1154a, 1154b) for adjusting the energy delivery capabilities of end effector (1146). For example, as shown in FIG. 15B, first ring electrodes (170a) may be selectively removed from first distal longitudinal segments (1154a) of first and third loop electrode assemblies (1150a, 1150c), and second ring electrodes (170b) may be selectively removed from first distal longitudinal segment (1154a) of second loop electrode assembly (1150b), such that first distal longitudinal segments (1154a) of first and third loop electrode assemblies (1150a, 1150c) are each disabled from applying RF energy at the first polarity and first distal longitudinal segment (1154a) of second loop electrode assembly (1150b) is disabled from applying RF energy at the second polarity. In this regard, cylinders (172) of such selectively removable ring electrodes (170a, 170b) may be selectively decoupled from the corresponding one or more wire(s), trace(s), and/or other conductive element(s) that electrically couple conductive cylinders (172) with RF generator (102) and may be slid (e.g., proximally) along the respective longitudinal segment(s) (1154a, 1154b) to remove the respective flexible support wire and/or sleeve (1162) from bores (174) of such cylinders (172). In other versions, cylinders (172) of such selectively removable ring electrodes (170a, 170b) may each have a split-ring configuration or any other suitable configuration for selectively attaching and removing ring electrodes (170a, 170b) to and from longitudinal segment(s) (1154a, 1154b).

### L. Alternative End Effector Having Triple Loop Electrode Assemblies with Dual Polarities and Removable Energy Application Elements

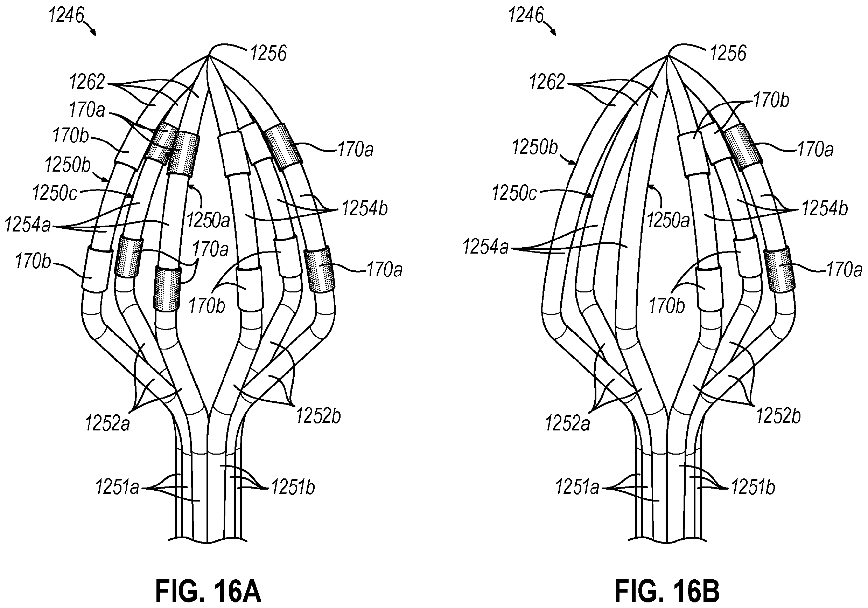

In some instances, it may be desirable to provide an elongate end effector for use with instrument (100) having additional energy delivery capabilities and/or additional energy delivery adjustment capabilities as compared to end effector (146). FIGS. 16A-16B show another exemplary end effector (1246) having such capabilities. End effector (1246) is substantially similar to end effector (146) except as otherwise described herein.

End effector (1246) of the present example has first, second, and third loop electrode assemblies (1250a, 1250b, 1250c) that may extend distally from a distal body portion of a corresponding energy catheter (not shown) along respective planes that may each be parallel to and/or aligned with the longitudinal axis of such a distal body portion and/or the longitudinal axis of shaft assembly (130). Each loop electrode assembly (1250a, 1250b, 1250c) includes first and second longitudinal segments (1251a, 1251b), first and second outwardly splayed segments (1252a, 1252b) coupled to distal ends of respective longitudinal segments (1251a, 1251b), first and second arcuate longitudinal segments (1254a, 1254b) extending distally from distal ends of respective outwardly splayed segments (1252a, 1252b), and a distal tip (1256) defined by distal ends of respective arcuate longitudinal segments (1254a, 1254b). In the example shown, first, second, and third loop electrode assemblies (1250a, 1250b, 1250c) each define a generally spearhead-like shape and intersect each other at the respective distal tips (1257).

Similar to loop electrode assemblies (150a, 150b), each loop electrode assembly (1250a, 1250b, 1250c) includes a flexible support wire (not shown), an insulative sleeve (1262), and a plurality of energy application elements in the form of first and second ring electrodes (170a, 170b). As shown in FIG. 16A, first ring electrodes (170a) are selectively positioned along first arcuate longitudinal segments (1254a) of first and third loop electrode assemblies (1250a, 1250c) and second arcuate longitudinal segment (1254b) of second loop electrode assembly (1250b). First arcuate longitudinal segments (1254a) of first and third loop electrode assemblies (1250a, 1250c) and second arcuate longitudinal segment (1254b) of second loop electrode assembly (1250b) are each thus configured to apply RF energy at a first polarity. Second ring electrodes (170b) are selectively positioned along second arcuate longitudinal segments (1254b) of first and third loop electrode assemblies (1250a, 1250c) and first arcuate longitudinal segment (1254a) of second loop electrode assembly (1250b). Second arcuate longitudinal segments (1254b) of first and third loop electrode assemblies (1250a, 1250c) and first arcuate longitudinal segment (1254a) of second loop electrode assembly (1250b) are each thus configured to apply RF energy at a second polarity.

In the example shown, at least a portion of first and/or second ring electrodes (170a, 170b) are selectively removable from the respective arcuate longitudinal segment(s) (1254a, 1254b) for adjusting the energy delivery capabilities of end effector (1246). For example, as shown in FIG. 16B, first ring electrodes (170a) may be selectively removed from first arcuate longitudinal segments (1254a) of first and third loop electrode assemblies (1250a, 1250c), and second ring electrodes (170b) may be selectively removed from first arcuate longitudinal segment (1254a) of second loop electrode assembly (1250b), such that first arcuate longitudinal segments (1254a) of first and third loop electrode assemblies (1250a, 1250c) are each disabled from applying RF energy at the first polarity and first arcuate longitudinal segment (1254a) of second loop electrode assembly (1250b) is disabled from applying RF energy at the second polarity. In this regard, cylinders (172) of such selectively removable ring electrodes (170a, 170b) may be selectively decoupled from the corresponding one or more wire(s), trace(s), and/or other conductive element(s) that electrically couple conductive cylinders (172) with RF generator (102) and may be slid (e.g., proximally) along the respective arcuate longitudinal segment(s) (1254a, 1254b) to remove the respective flexible support wire and/or sleeve (1262) from bores (174) of such cylinders (172). In other versions, cylinders (172) of such selectively removable ring electrodes (170a, 170b) may each have a split-ring configuration or any other suitable configuration for selectively attaching and removing ring electrodes (170a, 170b) to and from arcuate longitudinal segment(s) (1254a, 1254b).

### M. Alternative End Effector Having Double Angled Electrode Assemblies with Single Polarities

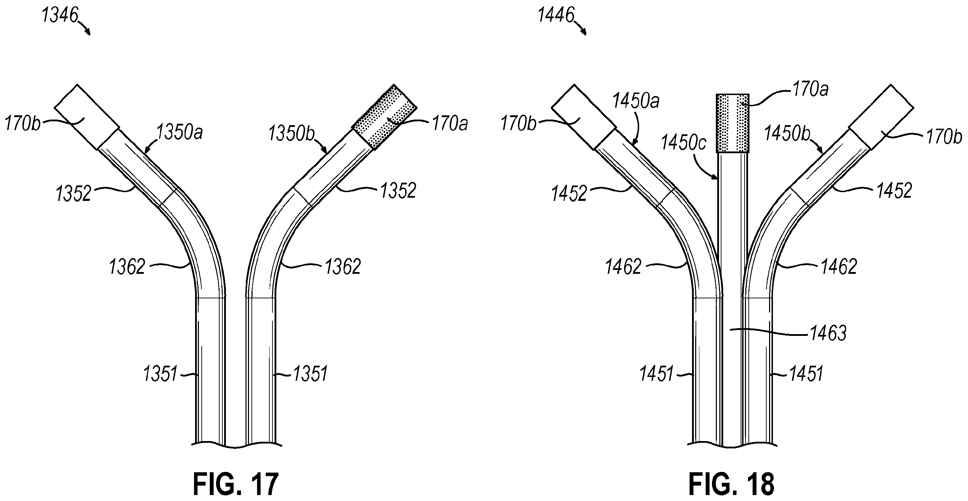

In some instances, it may be desirable to provide an elongate end effector for use with instrument (100) having different energy delivery capabilities and/or a flatter profile as compared to end effector (146). FIG. 17 shows another exemplary end effector (1346) having such capabilities. End effector (1346) is substantially similar to end effector (146) except as otherwise described herein.

End effector (1346) of the present example has a first and second angled (e.g., bent) electrode assemblies (1350a, 1350b) that may extend distally from a distal body portion of a corresponding energy catheter (not shown) along a plane that is parallel to and/or aligned with the longitudinal axis of such a distal body portion and/or the longitudinal axis of shaft assembly (130). Each angled electrode assembly (1350a, 1350b) includes a longitudinal segment (1351) and an outwardly splayed segment (1352) coupled to a distal end of the respective longitudinal segment (1351). In the present example, angled electrode assemblies (1350a, 1350b) collectively define a generally bident-like (e.g., "Y") shape.

Similar to loop electrode assemblies (150a, 150b), angled electrode assemblies (1350a, 1350b) may each include a flexible support wire (not shown) resiliently biased to collectively define the generally bident-like shape of angled electrode assemblies (1350a, 1350b), an insulative sleeve (1362), and at least one energy application element in the form of first and/or second ring electrodes (170a, 170b). In the example shown, first ring electrode (170a) is positioned at a distal end of outwardly splayed segment (1352) of second angled electrode assembly (1350b), and second ring electrode (170b) is positioned at a distal end of outwardly splayed segment (1352) of first angled electrode assembly (1350a), such that outwardly splayed segment (1352) of second angled electrode assembly (1350b) is configured to apply RF energy at a first polarity and outwardly splayed segment (1352) of first angled electrode assembly (1350a) is configured to apply RF energy at a second polarity. Due to this arrangement of ring electrodes (170a, 170b), the polarities of ring electrodes (170a, 170b) are distributed on opposing lateral sides relative to the longitudinal axis of shaft assembly (130). End effector (1346) may be used to apply bipolar RF energy to tissue as described herein (e.g., to provide ablation, electroporation, etc.).

### N. Alternative End Effector Having Double Angled Electrode Assemblies and Straight Electrode Assembly with Single Polarities

In some instances, it may be desirable to provide an elongate end effector for use with instrument (100) having different energy delivery capabilities and/or a flatter profile as compared to end effector (146). FIG. 18 shows another exemplary end effector (1446) having such capabilities. End effector (1446) is substantially similar to end effector (146) except as otherwise described herein.

End effector (1446) of the present example has a first and second angled electrode assemblies (1450a, 1450b) that may extend distally from a distal body portion of a corresponding energy catheter (not shown) along a plane that is parallel to and/or aligned with the longitudinal axis of such a distal body portion and/or the longitudinal axis of shaft assembly (130). Each angled electrode assembly (1450a, 1450b) includes a longitudinal segment (1451) and an outwardly splayed segment (1452) coupled to a distal end of the respective longitudinal segment (1451). In the present example, angled electrode assemblies (1450a, 1450b) collectively define a generally bident-like (e.g., "Y") shape.

In the example shown, end effector (1446) also has a straight electrode assembly (1450c) that may extend distally from the distal body portion of the corresponding energy catheter coaxially with the distal body portion and/or shaft assembly (130). As shown, straight electrode assembly (1450c) is coplanar with first and second angled electrode assemblies (1450a, 1450b), and a proximal portion of straight electrode assembly (1450c) is interposed between longitudinal segments (1451) of first and second angled electrode assemblies (1450a, 1450b). Angled electrode assemblies (1450a, 1450b) and straight electrode assembly (1450c) thus collectively define a generally trident-like shape.

Similar to loop electrode assemblies (150a, 150b), angled electrode assemblies (1450a, 1450b) may each include a flexible support wire (not shown) resiliently biased to define the generally bident shape of angled electrode assemblies (1450a, 1450b), an insulative sleeve (1462), and at least one energy application element in the form of second ring electrode (170b). In the example shown, second ring electrodes (170b) are positioned at distal ends of outwardly splayed segments (1452) of first and second angled electrode assemblies (1450a, 1450b), such that outwardly splayed segments (1452) of first and second angled electrode assemblies (1450a, 1450b) are each configured to apply RF energy at a second polarity. Straight electrode assembly (1450c) may likewise include a flexible support wire (not shown) resiliently biased to define the generally elongate shape of straight electrode assembly (1450c), an insulative sleeve (1463), and at least one energy application element in the form of first ring electrode (170a). In this manner, straight electrode assembly (1450c) may be configured to apply RF energy at a first polarity. Thus, angled electrode assemblies (1450a, 1450b) may each cooperate with straight electrode assembly (1450c) to apply bipolar RF energy to tissue.

### III. Exemplary Method of Ablating Posterior Nasal Nerve

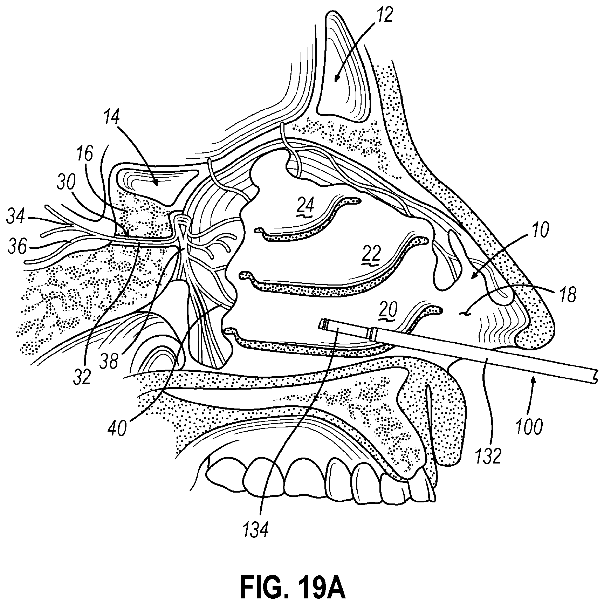

Having described exemplary features of instrument (100) above, an exemplary method of performing an ablation on a posterior nasal nerve (40) of a patient with instrument (100) will now be described in connection with FIGS. 19A-19B. While the exemplary method is showing being performed with instrument (100) equipped with energy catheter (140), it will be appreciated that similar methods may be performed using instrument (100) equipped with any of energy catheters (240, 340, 440, 540, 640) or end effectors (746, 846, 946, 1046, 1146, 1246, 1346, 1446). Additionally, while instrument (100) is shown and described for treating a posterior nasal nerve, it will be appreciated that instrument (100) may be employed in various other surgical applications for ablating other nerves or anatomical structures within the nasal cavity (10), or for ablating tissues in various other anatomical regions of a patient. For instance, the teachings herein may be combined with at least some of the teachings of U.S. Pat. Pub. No. 2019/0374280, entitled "Apparatus and Method for Performing Vidian Neurectomy Procedure," published December 12, 2019; and/or with at least some of the teachings of U.S. Pat. Pub. No. 2022-0087739 (U.S. Pat. App. No. 17/404,088), entitled "ENT Instrument with Expandable Ablation Feature," filed August 17, 2021.

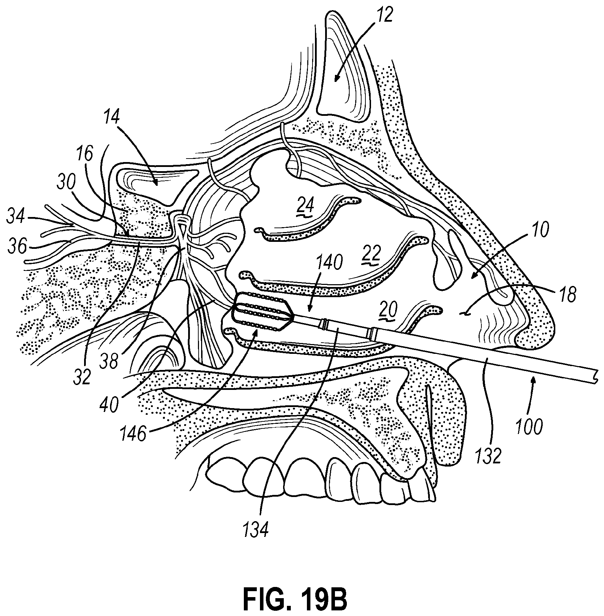

As shown in FIG. 19A, the distal end of instrument (100) is inserted into the nasal cavity (10) and is toward the posterior ends of the inferior and middle turbinates (20, 22), which may be performed under visualization provided by an endoscope (not shown), for example. Upon reaching a target site of the nasal wall (18) in which a target portion of the posterior nasal nerve (40) resides, the operator advances slider (122) distally to thereby extend and expand loop electrode assemblies (150a, 150b) of end effector (146), as shown in FIG. 19B, to urge electrode assemblies (150a, 150b) and, more particularly, ring electrodes (170a, 170b) into electrical contact with a target portion of posterior nasal nerve (40). Ring electrodes (170a, 170b) are then energized with bipolar RF energy to thereby ablate the targeted portion of posterior nasal nerve (40).

### V. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility or by a user immediately prior to a procedure. Those skilled in the art will appreciate that reconditioning of a device may utilize a variety of techniques for disassembly, cleaning/replacement, and reassembly.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art. The scope of the invention is defined by the appended claims. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument (100) comprising:
(a) a sheath configured to be inserted into a nasal cavity of a patient's head; and
(b) an energy catheter (140) disposed within the sheath, wherein the energy catheter includes:
(i) a shaft (130) extending along a longitudinal axis, and
(ii) an elongate, expandable end effector (146) extending distally from the shaft and configured to selectively expand radially outwardly from a non-expanded state to an expanded state, wherein the end effector includes a plurality of loop-shaped energy members (150a, 150b), each of the plurality of loop-shaped energy members comprising:
(A) at least one flexible wire (160), wherein the at least one flexible wire is resiliently biased to define a bulbous shape of the loop shaped energy member, and
(B) a plurality of electrodes (170a, 170b) disposed along the at least one flexible wire
wherein each of the plurality of loop-shaped energy members extends along a respective plane, wherein each respective plane is parallel to and laterally offset from the longitudinal axis;
wherein the plurality of loop-shaped energy members overlap each other;
wherein the energy catheter is selectively translatable relative to the sheath between a proximal retracted position in which the end effector is housed coaxially within the sheath to thereby prevent the end effector from radially expanding from the non-expanded state to the expanded state, and a distal extended position in which the end effector is exposed from the sheath to thereby permit the end effector to expand from the non-expanded state to the expanded state for contacting tissue in the nasal cavity of the patient's head,
wherein when the end effector is in the expanded state, the end effector has a width and a length greater than the width.

2. The surgical instrument of claim **1,** wherein the at least one flexible wire comprises an electrically conductive material.

3. The surgical instrument of claim **1,** wherein the at least one flexible wire comprises a resiliently biased material.

4. The surgical instrument of claim **1,** wherein the end effector is configured to resiliently expand radially from the non-expanded state to the expanded state in response to the energy catheter translating from the proximal retracted position to the distal extended position.

5. The surgical instrument of claim **1,** wherein each loop-shaped energy member further includes at least one electrically insulative layer (162).

6. The surgical instrument of claim 5, wherein the at least one electrically insulative layer extends coaxially about the at least one flexible wire.

7. The surgical instrument of claim 5, wherein the at least one electrically insulative layer extends between adjacent electrodes of the plurality of electrodes.

8. The surgical instrument of claim 5, wherein the at least one electrically insulative layer is presented by a portion of an outer surface of at least one electrode of the plurality of electrodes.

9. The surgical instrument of claim 8, wherein the at least one electrically insulative layer includes an electrically insulative coating.

10. The surgical instrument of claim 1, wherein at least one electrode of the plurality of electrodes is selectively removable from the at least one flexible wire.

11. The surgical instrument of claim 1, wherein the plurality of loop-shaped energy members are positioned about the longitudinal axis.

## Patentansprüche

1. Chirurgisches Instrument (100), umfassend:
(a) eine Hülse, die ausgestaltet ist, um in eine Nasenhöhle des Kopfes eines Patienten eingeführt zu werden; und
(b) einen Energiekatheter (140), der innerhalb der Hülse angeordnet ist, wobei der Energiekatheter einschließt:
(i) einen Schaft (130), der sich entlang einer Längsachse erstreckt, und
(ii) einen länglichen expandierbaren Endeffektor (146), der dich distal von dem Schaft erstreckt und ausgestaltet ist, um selektiv radial auswärts aus einem nicht-expandierten Zustand zu einem expandierten Zustand zu expandieren, wobei der Endeffektor eine Vielzahl von schlaufenförmigen Energieelementen (150a, 150b) einschließt, wobei jedes der Vielzahl von schlaufenförmigen Energieelementen umfasst:
(A) mindestens einen flexiblen Draht (160), wobei der mindestens eine flexible Draht elastisch vorgespannt ist, um eine knollige Form des schlaufenförmigen Energieelements zu definieren, und
(B) eine Vielzahl von Elektroden (170a, 170b), die entlang des mindestens einen flexiblen Drahts angeordnet ist,
wobei jedes von der Vielzahl der schlaufenförmigen Energieelemente sich entlang einer jeweiligen Ebene erstreckt, wobei jede jeweilige Ebene parallel zu und lateral versetzt von der Längsachse ist;
wobei die Vielzahl der schlaufenförmigen Energieelemente einander überlappt;
wobei der Energiekatheter selektiv relativ zu der Hülse zwischen einer proximalen zurückgezogenen Position, in welcher der Endeffektor koaxial innerhalb der Hülse untergebracht ist, um dadurch zu verhindern, dass der Endeffektor aus dem nicht-expandierten Zustand radial zu dem expandierten Zustand expandiert, und einer distalen ausgefahrenen Position translatierbar ist, in welcher der Endeffektor von der Hülse exponiert ist, um dadurch zuzulassen, dass der Endeffektor aus dem nicht-expandierten Zustand zu dem expandierten Zustand expandiert, um Gewebe in der Nasenhöhle des Kopfes eines Patienten zu kontaktieren,
wobei der Endeffektor, wenn sich der Endeffektor in dem expandierten Zustand befindet, eine Breite und eine Länge, welche größer als die Breite ist, aufweist.

2. Chirurgisches Instrument nach Anspruch 1, wobei der mindestens eine flexible Draht ein elektrisch leitfähiges Material umfasst.

3. Chirurgisches Instrument nach Anspruch 1, wobei der mindestens eine flexible Draht ein elastisch vorgespanntes Material umfasst.

4. Chirurgisches Instrument nach Anspruch 1, wobei der Endeffektor ausgestaltet ist, um in Reaktion darauf, dass der Energiekatheter aus der proximalen zurückgezogenen Position zu der distalen ausgefahrenen Position translatiert, aus dem nicht-expandierten Zustand radial elastisch zu dem expandierten Zustand zu expandieren.

5. Chirurgisches Instrument nach Anspruch 1, wobei jedes schlaufenförmige Energieelement ferner mindestens eine elektrisch isolierende Schicht (162) einschließt.

6. Chirurgisches Instrument nach Anspruch 5, wobei die mindestens eine elektrisch isolierende Schicht sich koaxial um den mindestens einen flexiblen Draht erstreckt.

7. Chirurgisches Instrument nach Anspruch **5,** wobei die mindestens eine elektrisch isolierende Schicht sich zwischen benachbarten Elektroden der Vielzahl von Elektroden erstreckt.

8. Chirurgisches Instrument nach Anspruch 5, wobei die mindestens eine elektrisch isolierende Schicht durch einen Abschnitt einer Außenoberfläche von mindestens einer Elektrode der Vielzahl von Elektroden dargestellt wird.

9. Chirurgisches Instrument nach Anspruch 8, wobei die mindestens eine elektrisch isolierende Schicht eine elektrisch isolierende Beschichtung einschließt.

10. Chirurgisches Instrument nach Anspruch 1, wobei mindestens eine Elektrode der Vielzahl von Elektroden selektiv von dem mindestens einen flexiblen Draht entfernbar ist.

11. Chirurgisches Instrument nach Anspruch 1, wobei die Vielzahl der schlaufenförmigen Energieelemente um die Längsachse positioniert ist.

## Revendications

1. Instrument chirurgical (100) comprenant :
(a) une gaine configurée pour être insérée dans la cavité nasale de la tête d'un patient ; et
(b) un cathéter d'énergie (140) disposé à l'intérieur de la gaine, le cathéter d'énergie comprenant :
(i) une tige (130) s'étendant le long d'un axe longitudinal, et
(ii) un effecteur d'extrémité allongé et déployable (146) s'étendant de manière distale à partir de la tige, et configuré pour s'étendre de manière sélective radialement vers l'extérieur depuis un état non déployé vers un état déployé, où l'effecteur d'extrémité comprend une pluralité d'éléments énergétiques en forme de boucle (150a, 150b), chacun de la pluralité d'éléments énergétiques en forme de boucle comprenant :
(A) au moins un fil flexible (160), l'au moins un fil flexible étant sollicité de manière élastique pour définir une forme bulbeuse de l'élément énergétique en forme de boucle, et
(B) une pluralité d'électrodes (170a, 170b) disposées le long de l'au moins un fil flexible
où chacun de la pluralité d'éléments énergétiques en forme de boucle s'étend le long d'un plan respectif, chaque plan respectif étant parallèle à l'axe longitudinal et décalé latéralement par rapport à celui-ci ;
où la pluralité d'éléments énergétiques en forme de boucle se chevauchent les uns les autres ;
où le cathéter énergétique est translatable de manière sélective par rapport à la gaine entre une position proximale rétractée dans laquelle l'effecteur d'extrémité est logé coaxialement à l'intérieur de la gaine afin d'empêcher ainsi l'effecteur d'extrémité de se dilater radialement de l'état non déployé à l'état déployé, et une position distale étendue dans laquelle l'effecteur d'extrémité est exposé à partir de la gaine afin de permettre ainsi à l'effecteur d'extrémité de se déployer de l'état non déployé à l'état déployé pour entrer en contact avec les tissus de la cavité nasale de la tête du patient,
où, lorsque l'effecteur d'extrémité est à l'état déployé, l'effecteur d'extrémité a une certaine largeur et une longueur supérieure à la largeur.

2. Instrument chirurgical selon la revendication 1, dans lequel l'au moins un fil flexible comprend un matériau électriquement conducteur.

3. Instrument chirurgical selon la revendication 1, dans lequel l'au moins un fil flexible comprend un matériau à sollicitation élastique.

4. Instrument chirurgical selon la revendication 1, dans lequel l'effecteur d'extrémité est configuré pour se déployer élastiquement radialement de l'état non déployé à l'état déployé en réponse au déplacement du cathéter énergétique de la position proximale rétractée à la position distale étendue.

5. Instrument chirurgical selon la revendication 1, dans lequel chaque élément énergétique en forme de boucle comprend en outre au moins une couche électriquement isolante (162).

6. Instrument chirurgical selon la revendication 5, dans lequel l'au moins une couche électriquement isolante s'étend coaxialement autour de l'au moins un fil flexible.

7. Instrument chirurgical selon la revendication 5, dans lequel l'au moins une couche électriquement isolante s'étend entre des électrodes adjacentes de la pluralité d'électrodes.

8. Instrument chirurgical selon la revendication 5, dans lequel l'au moins une couche électriquement isolante est présentée par une partie d'une surface extérieure d'au moins une électrode de la pluralité d'électrodes.

9. Instrument chirurgical selon la revendication 8, dans lequel l'au moins une couche électriquement isolante comprend un revêtement électriquement isolant.

10. Instrument chirurgical selon la revendication 1, dans lequel au moins une électrode parmi la pluralité d'électrodes peut être retirée de manière sélective de l'au moins un fil flexible.

11. Instrument chirurgical selon la revendication 1, dans lequel la pluralité d'éléments énergétiques en forme de boucle sont positionnés autour de l'axe longitudinal.
